Europäisches Patentamt

European Patent Office

Office européen des brevets

(11) Publication number: **0 401 379 B1**

# EUROPEAN PATENT SPECIFICATION

(45) Date of publication of patent specification: **15.06.94**   (51) Int. Cl.5: **A61K 37/02**

(21) Application number: **89913181.7**

(22) Date of filing: **02.12.89**

(86) International application number:
**PCT/JP89/01212**

(87) International publication number:
**WO 90/06127 (14.06.90 90/14)**

(54) **STABILIZED COMPOSITION OF INTERLEUKIN-1-g(b).**

(30) Priority: **06.12.88 JP 309264/88**

(43) Date of publication of application:
**12.12.90 Bulletin 90/50**

(45) Publication of the grant of the patent:
**15.06.94 Bulletin 94/24**

(84) Designated Contracting States:
**CH DE ES FR GB IT LI NL SE**

(56) References cited:
**EP-A- 0 158 487      EP-A- 0 237 967
EP-A- 0 268 110      EP-A- 0 352 816
EP-A- 0 360 226      EP-A- 0 391 444
WO-A-88/03031        JP-A- 6 197 229
JP-A-58 206 513      JP-A-61 293 926**

(73) Proprietor: **OTSUKA PHARMACEUTICAL CO., LTD.
9, Kandatsukasa-cho 2-chome
Chiyoda-ku Tokyo 101(JP)**

(72) Inventor: **SUGAHARA, Yuji, 1-36, Aza
Nakasenakanokoshi
Nakakirai, Matsushige-cho
Itano-gun, Tokushima 771-02(JP)**
Inventor: **KAISE, Hirotsugu
28-15, Aza Nakazao, Shinkirai,
Kitajima-cho
Itano-gun, Tokushima 771-02(JP)**

(74) Representative: **Hansen, Bernd, Dr.rer.nat. et al
Hoffmann, Eitle & Partner
Patentanwälte
Postfach 81 04 20
D-81904 München (DE)**

Rank Xerox (UK) Business Services
(3.10/3.09/3.3.3)

EP 0 401 379 B1

## Description

This invention relates to a novel composition containing an interleukin-1$\beta$ (IL-1$\beta$) active substance stabilized therein.

It is known that IL-1$\beta$ is produced not only by macrophages or monocytes but also by many different kinds of cells and exhibits diverse biological activities [see e.g. Taisha (Metabolism and Disease), Vol. 23, Special Issue 86, "Immunity", pages 97-104 (1986); Medical Immunology, Vol. 12, No. 6, pages 753-760 (1986)].

Based on the above activities, it is expected that said IL-1$\beta$ should be applicable as a drug. For instance, the present applicant has previously revealed that IL-1$\beta$ and derivatives thereof are effective in various pharmaceutical uses [see laid-open European Patent Application EP 0237967 A2].

EP-A2-0237967 discloses a composition containing the interleukin-1$\beta$ active substance [Ser[71]]IL-$\beta$ and human serum albumin.

WO-A-8803031 refers to a therapeutic composition for the treatment of arthritis or inflammation comprising an effective amount of IL-1$\beta$ and a physiologically acceptable carrier or diluent. As appropriate diluents there are mentioned neutral buffered saline or saline mixed with conspecific serum albumin.

Meanwhile, in applying a substance as a drug, it is of course required that the active ingredient should not be degraded with time but remain stable in its ordinary pharmaceutical forms and under storage conditions. In the case of the above-mentioned IL-1$\beta$, it is also naturally required that it should have the performance characteristics just mentioned above. In particular in the case of a homogeneous sample highly purified so that it can be applied clinically, much care is required in handling it from the stability viewpoint, and various restrictions are encountered in maintaining its activities. Thus the development of or improvement in a pharmaceutical composition in which IL-1$\beta$ is stabilized and can remain more stable under freezing or lyophilization treatment conditions and various storage conditions (e.g. temperature, period) is desired in the art.

While the IL-1$\beta$ active substance has potent pharmacological activities and therefore is used in very small amounts, it is likely to adsorb on the container wall. Since this problem of adsorption becomes serious with decreases in the content of IL-B active substance, it becomes necessary to prevent the adsorption. The IL-1$\beta$ active substance is also unstable; the instability increases with decreases in the content of the active ingredient.

EP-A-0158487 discloses that IL-2 can be effectively stabilized by adjusting the pH in the presence of human albumin (B) alone or in combination with a reducing agent.

EP-A-268110 discloses a pharmaceutical composition comprising a recombinant interleukin-2 protein dissolved in an inert carrier medium comprising as stabilizer an effective amount of one or more biocompatible non-ionic polymeric detergents.

JP-A-61097229 discloses the effective use of polyethylene glycol, proteins, sugars, amino acids, inorganic acid salts, organic acid salts and sulfur containing-reducing agents for stabilization of erythropoietin.

The above references do not disclose the stabilization of interleukin-1$\beta$ compositions.

It is an object of the invention to solve the above problems and to provide a composition containing an IL-1$\beta$ active substance stabilized therein which is particularly suitable for clinical use and further to provide an isotonic composition containing the above-mentioned IL-1$\beta$ active substance stabilized therein.

The present inventors conducted intensive investigations for the above purposes and found that human serum albumin and a saccharide, when used in combination with an Il-1$\beta$ active substance, display an adsorption preventing effect and a stabilizing effect, whereby the activities of the IL-1$\beta$ active substance are markedly stabilized. This finding has now led to completion of the present invention.

The present invention thus provides a stabilized IL-1$\beta$ composition comprising human serum albumin and a saccharide together with an IL-1$\beta$ active substance.

The IL-1$\beta$ active substance to be used as an active ingredient in the composition according to the invention includes IL-1$\beta$ and derivatives thereof. As such active ingredients, there may be mentioned, for example, those polypeptides that are described in the applicant's earlier laid-open European patent application (EP 0237967 A2) and equivalents to such polypeptides. Said polypeptides are characterized in that they have an amino acid sequence which fills at least one of the requirements a) to d) mentioned below:

2

a) That, in the amino acid sequence of IL-1$\beta$ represented by the formula (A):

```
Ala-Pro-Val-Arg-Ser-Leu-Asn-Cys-Thr-Leu-Arg-

Asp-Ser-Gln-Gln-Lys-Ser-Leu-Val-Met-Ser-Gly-

Pro-Tyr-Glu-Leu-Lys-Ala-Leu-His-Leu-Gln-Gly-

Gln-Asp-Met-Glu-Gln-Gln-Val-Val-Phe-Ser-Met-

Ser-Phe-Val-Gln-Gly-Glu-Glu-Ser-Asn-Asp-Lys-

Ile-Pro-Val-Ala-Leu-Gly-Leu-Lys-Glu-Lys-Asn-

Leu-Tyr-Leu-Ser-Cys-Val-Leu-Lys-Asp-Asp-Lys-

Pro-Thr-Leu-Gln-Leu-Glu-Ser-Val-Asp-Pro-Lys-

Asn-Tyr-Pro-Lys-Lys-Lys-Met-Glu-Lys-Arg-Phe-

Val-Phe-Asn-Lys-Ile-Glu-Ile-Asn-Asn-Lys-Leu-

Glu-Phe-Glu-Ser-Ala-Gln-Phe-Pro-Asn-Trp-Tyr-

Ile-Ser-Thr-Ser-Gln-Ala-Glu-Asn-Met-Pro-Val-

Phe-Leu-Gly-Gly-Thr-Lys-Gly-Gly-Gln-Asp-Ile-
```

at least one amino acid residue selected from the group consisting of 1-position Ala, 3-position Val, 4-position Arg, 5-position Ser, 8-position Cys, 11-position Arg, 30-position His, 71-position Cys, 93-position Lys, 97-position Lys, 98-position Arg, 99-position Phe, 103-position Lys, 120-position Trp, 121-position Tyr and 153-position Ser is deficient or replaced by another amino acid residue;

b) That, in the sequence of formula (A), the amino acid sequence of 1-position Ala to 9-position Thr, or at least one amino acid residue thereof is deficient [excluding those cases where at least one amino acid residue selected from the group consisting of 1-position Ala, 3-position Val, 4-position Arg, 5-position Ser and 8-position Cys is deficient as described above in a)];

c) That, in the sequence of formula (A), the amino acid sequence of 103-position Lys to 153-position Ser, or at least one amino acid residue thereof is deficient (excluding those cases where at least one amino acid residue selected from the group consisting of 103-position Lys, 120-position Trp, 121-position Tyr and 153-position Ser is deficient as described above in a)];

d) That an amino acid sequence of 1'-position Met to 116'-position Asp represented by the formula (B) given below or a partial amino acid sequence on the C terminal side thereof is attached to the N terminus of the above formula (A):

Formula (B):

```
Met-Ala-Glu-Val-Pro-Glu-Leu-Ala-Ser-Glu-Met-

Met-Ala-Tyr-Tyr-Ser-Gly-Asn-Glu-Asp-Asp-Leu-

Phe-Phe-Glu-Ala-Asp-Gly-Pro-Lys-Gln-Met-Lys-

Cys-Ser-Phe-Gln-Asp-Leu-Asp-Leu-Cys-Pro-Leu-

Asp-Gly-Gly-Ile-Gln-Leu-Arg-Ile-Ser-Asp-His-

His-Tyr-Ser-Lys-Gly-Phe-Arg-Gln-Ala-Ala-Ser-

Val-Val-Val-Ala-Met-Asp-Lys-Leu-Arg-Lys-Met-

Leu-Val-Pro-Cys-Pro-Gln-Thr-Phe-Gln-Glu-Asn-

Asp-Leu-Ser-Thr-Phe-Phe-Pro-Phe-Ile-Phe-Glu-

Glu-Glu-Pro-Ile-Phe-Phe-Asp-Thr-Trp-Asp-Asn-

Glu-Ala-Tyr-Val-His-Asp
```

Amino acids and polypeptides are herein referred to by symbols according to the nonmenclature or the rules recommended by IUPAC or IUPAC-IUB or symbols conventionally used in the art. The same applies to the symbols of nucleic acids as used in base sequences. The number or position of each amino acid is always indicated in accordance with the amino acid sequence of IL-1$\beta$, namely the sequence of the above formula (A), unless otherwise stated, even in the case where there is (are) a deletion(s) and/or an attachment(s). The number with prime (') representing the position of amino acid indicates the corresponding position in the amino acid sequence of formula (B).

In the following, detailed mention is made of the above polypeptides (IL-1$\beta$ and derivatives thereof).

The IL-1$\beta$ derivatives mentioned above are polypeptides comprising an amino acid sequence which fulfills one or a combination of two or more of the requirements a) to d) in the amino acid sequence of IL-1$\beta$ as represented by the above formula (A). Preferred derivatives are those having an amino acid sequence which fulfills at least one of the above requirements a) to c) and those having an amino acid sequence which fulfills the requirement d) as well as at least one of the requirements a) to c).

Preferred specific examples of the polypeptides, which are IL-1$\beta$ derivatives, are as follows:

1) A polypeptide in which at least the 1-position Ala is deficient or replaced by a different amino acid residue.

2) A polypeptide in which at least the 3-position Val is deficient or replaced by a different amino acid residue.

3) A polypeptide in which at least the 4-position Arg is deficient or replaced by a different amino acid residue.

4) A polypeptide in which at least the 5-position Ser is deficient or replaced by a different amino acid residue.

5) A polypeptide in which at least the 8-position Cys is deficient or replaced by a different amino acid residue.

6) A polypeptide in which at least the 11-position Arg is deficient or replaced by a different amino acid residue.

7) A polypeptide in which at least the 30-position His is deficient or replaced by a different amino acid residue.

8) A polypeptide in which at least the 71-position Cys is deficient or replaced by a different amino acid residue.

9) A polypeptide in which at least the 93-position Lys is deficient or replaced by a different amino acid residue.

10) A polypeptide in which at least the 97-position Lys is deficient or replaced by a different amino acid residue.

11) A polypeptide in which at least the 98-position Arg is deficient or replaced by a different amino acid residue.

12) A polypeptide in which at least the 99-position Phe is deficient or replaced by a different amino acid residue.

13) A polypeptide in which at least the 103-position Lys is deficient or replaced by a different amino acid residue.

14) A polypeptide in which at least the 120-position Trp is deficient or replaced by a different amino acid residue.

15) A polypeptide in which at least the 121-position Tyr is deficient or replaced by a different amino acid residue.

16) A polypeptide in which at least the 153-position Ser is deficient or replaced by a different amino acid residue.

17) A polypeptide in which at least the amino acid sequence of 1-position Ala to 3-position Val, the amino acid sequence of 1-position Ala to 6-position Leu or the amino acid sequence of 1-position Ala to 9-position Thr is deficient.

18) A polypeptide in which at least the amino acid sequence of 151-position Val to 153-position Ser, the amino acid sequence of 149-position Gln to 153-position Ser, the amino acid sequence of 145-position Asp to 153-position Ser, the amino acid sequence of 141-position Gln to 153-position Ser, the amino acid sequence of 121-position Tyr to 153-position Ser or the amino acid sequence of 103-position Lys to 153-position Ser is deficient.

19) A polypeptide which has at least an amino acid residue at the N terminus of formula (A).

20) A polypeptide which at least has, at the N-terminus of formula (A), the amino acid sequence of 112'-position Ala to 116'-position Asp, the amino acid sequence of 77'-position Met to 116'-position Asp, the amino acid sequence of 71'-position Met to 116'-position Asp, the amino acid sequence of 32'-position Met to 116'-position Ser or the amino acid sequence of 1'-position Met to 116'-position Asp, which are present in the amino acid sequence of formula (B).

The above IL-1$\beta$ derivatives include polypeptides having an amino acid sequence in which a specific amino acid residue at a specific position is replaced by another amino acid residue or in which an amino acid residue is attached at a specific position. The amino acid residue for replacement and attachment may be any $\alpha$-amino acid residue that is a constituent of human proteins, and is preferably a neutral amino acid residue. Since, however, Cys may form, owing to its SH group, an intramolecular or intermolecular disulfide bond, said amino acid residue should preferably be any amino acid residue such as mentioned above but Cys. As particularly preferred examples, there may be mentioned Gly, Lys, Gln or Asp for the 4-position Arg, Ser or Ala for the 8-position Cys, Gln for the 11-position Arg, Tyr for the 30-position His, Ser, Ala or Val for the 71-position Cys, Leu or Asp for the 93-position Lys, Leu for the 98-position Arg, Gln for the 103-position Lys, Arg for the 120-position Trp, Gln for the 121-position Tyr, and Met, Leu, Arg or Asp for the attachment to the N terminus.

Such IL-1$\beta$ derivatives and IL-1$\beta$ have, for example, LAF activity; tumor cell growth inhibiting activity (GIF activity), namely ability to inhibit the growth of tumor cells specifically; ability to promote the production of various cytokines such as colony stimulating factor (CSF), interferons (IFNs), interleukin-2 (IL-2) and interleukin-3 (IL-3), namely ability to act on human cells, for instance, and markedly promote the production of such cytokines; antiinflammatory activity, particularly ability to effectively prevent the progress of arthritis in arthritis model animals, for instance; and radiation damage-preventing effect, namely ability to prevent those damages to or severe side effects on living organisms that are due to whole body irradiation in the case of bone marrow transplantation or irradiation for the treatment of cancer and the like, or are encountered in the case of a radiation accident. The IL-1$\beta$ derivatives mentioned above are superior in at least one of the activities mentioned above and/or in low toxicity and little side effect. Therefore the above-mentioned IL-1$\beta$ derivatives and IL-1$\beta$ are useful as such drugs as immune system stimulating agents for promoting antibody production or for potentiating vaccines, antitumor agents, agents for promoting production of cytokines such as CSF, IL-2 and IL-3, antiinflammatory agents and radiation damage preventing agents, among others.

In particular, the new finding that the above-mentioned IL-1β and derivatives thereof are markedly effective against inflammation, for example arthritis, is a surprising effect, contrary to the fact that IL-1 has been said to mediate inflammation and participate in the induction thereof. Furthermore, the IL-1β derivatives are superior in at least one of the above-mentioned activities and/or in low toxicity and little side effect.

In particular, the above-mentioned IL-1β and derivatives thereof are effective as CSF production promoters and, when they are administered to humans, can be effectively restored granulocytopenia due to depressed myelopoiesis following cancer chemotherapy or radiation therapy (therapeutic agents for granulocytopenia) without any risk of viral infection or antigen-antibody reaction. Owing to their intrinsic CSF production promoting activity, the above-mentioned CSF production promoters can effectively be used also as prophylactic or therapeutic agents for various diseases through the action of CSF. For example, in view of the fact that CSF can promote the functions of granulocytes and macrophages [Lopez, A. F. et al., J. Immunol., 131, 2983 (1983); Handam, E. et al., ibid., 122, 1134 (1979); and Vadas, M. A. et al., ibid., 130, 795 (1983)] and is expected to be clinically applicable as a prophylactic and therapeutic agent for various infectious diseases, the above-mentioned CSF production promoters are expected to be clinically applicable in a similar manner.

In particular, the research and development of new agents for combating with the so-called opportunistic infection or terminal infection, which is induced in compromised hosts, in which the defense mechanism is depressed or in disorder, by microorganisms that are usually harmless but can become pathogenic in such hosts, is earnestly desired since the antibiotics currently in use can hardly be satisfactorily effective against such opportunistic infection-inducing pathogens (pathogenic microorganisms) of clinical importance, inclusive of gram-negative rods such as Pseudomonas and Serratia, viruses such as herpes simplex virus (HSV), varicella zoster virus (VZV) and cytomegalovirus (CMV), fungi such as Candida albicans, Aspergillus fumigatus and Nocardia asteroidea, protozoa such as Pneumocystis carinii and Toxoplasma gondii, etc. The IL-1β derivatives are useful also as prophylactic and therapeutic agents for such opportunistic infection, in particular as prophylactic and therapeutic agents for various infectious diseases following administration of anti-cancer agents, namely chemotherapy of acute leukemia, or bone marrow transplantation, where such opportunistic infection is encountered with high incidences, such as candidiasis, cryptococcosis, aspergillosis, mucormycosis, chromomycosis, viral infections, cytomegaloviral pneumonia, and complications of these.

Furthermore, in addition to their pharmaceutical use, IL-1β and derivatives thereof can be used very effectively in the in vitro production of various useful cytokines from cell lines, owing to their cytokine production promoting activity. Such production of natural type cytokines from cell lines is noticed particularly in the case of those cytokines that are glycoproteins, and can afford useful cytokines in large amounts and in an efficient manner.

Among the IL-1β derivatives mentioned above, those in which at least the 71-position Cys is replaced or deficient, in particular those in which the above-mentioned Cys is replaced by a different amino acid residue, for example Ser, Ala or Val, show remarkable activity.

Those IL-1β derivatives in which at least the 4-position Arg, 93-position Lys and 8-position Cys are replaced or deficient, and those derivatives in which at least one amino acid residue at position 103 or at any subsequent position is deficient are all characterized in that their prostaglandin E (PGE) production promoting activity is weak and therefore their pyrogenicity and other side effects and toxicity are of a low degree. Furthermore, those derivatives in which at least the 4-position Arg or 93-position Lys is replaced or deficient are characterized in that their CSF production promoting activity and antiinflammatory activity are more potent as compared with the GIF and LAF activities.

Furthermore, among the above-mentioned derivatives, those in which at least a specific amino acid residue or polypeptide is attached to the N terminus of formula (A) are characterized in that their CSF production promoting activity and antiinflammatory activity are higher as compared with the GIF activity and LAF activity and, in addition, low in toxicity and long lasting activity; these respects are advantageous for their use as drugs, in particular in the form of oral preparations or suppositories.

Furthermore, the above-mentioned derivatives, in particular those in which at least the 8-position Cys and/or 71-position Cys is replaced or deficient, preferably those in which said Cys is replaced by a different amino acid residue, for example Ser, Ala or Val, are superior in affinity for IL-1 receptors under various conditions.

Among the above-mentioned derivatives, those which are free of Cys or contain in their molecule a less number of Cys residues than IL-1β are preferred considering that there is no unnecessary formation of an intramolecular or intermolecular bond due to the SH group of Cys.

6

The specific polypeptides mentioned above, namely IL-1β and derivatives thereof, can be produced, for example by using genetic engineering techniques. Thus, use is made of genes coding for the above-mentioned specific polypeptides. The polypeptides can be produced by inserting said genes into vectors which can be carried by microorganisms and allowing replication, transcription and translation of the genes to occur in the cells of said microorganisms. This method is advantageous in that mass production is possible.

The genes to be used in the above method may be totally synthesized in the manner of chemical synthesis of nucleic acids by a conventional method, for example the phosphite triester method [Nature, 310, 105 (1984)]. It is simple and easy, however, to synthesize or produce them utilizing a gene coding for IL-1β or a precursor thereto, for example by modifying said gene in a conventional manner including the above-mentioned means of chemical synthesis to give nucleic acid sequences coding for the specific amino acid sequences mentioned above.

The gene coding for IL-1β or a precursor thereto is known. The present applicant has succeeded in obtaining the gene coding for IL-1β and producing IL-1β using said gene by the genetic engineering techniques as described in its earlier application (Japanese Patent Application No. 60-138281, Japanese Kokai Tokkyo Koho No. 62-174022).

The above nucleic acid (base) sequence modification can be carried out using known methods so that the amino acid sequence of the desired polypeptide can be obtained. [For the genetic engineering techniques, see, for example, Molecular Cloning, Cold Spring Harbor Laboratory (1982).]

Thus, for instance, such conventional means as treatment with various enzymes such as restriction enzymes, DNA ligase, polynucleotide kinase and DNA polymerase, which are used for DNA cleavage, ligation, phosphorylation or other purposes, can be employed and such enzymes are readily available as commercial products. The isolation and purification of each gene or nucleic acid in each of these steps also can be accomplished by conventional methods, for example by agarose electrophoresis. For the replication of the gene obtained, use may be made of the ordinary method comprising the use of a vector, which method will partly be mentioned further hereinafter. DNA fragments coding for the desired amino acid sequence as well as synthetic linkers can be readily produced by the above-mentioned chemical synthesis. The codon or codons corresponding to each desired amino acid in the above-mentioned fragments or linkers are by themselves known, and the selection of codon is decided arbitrarily in a conventional manner, for example, taking into account the frequency of use of the codon in question in the host employed [Nucl. Acids Res., 9, 43-74 (1981)]. For partial modification of a codon in those nucleic acid sequences, site-specific mutagenesis in which a primer consisting of a synthetic oligonucleotide (about 15- to 30-mer) coding for the desired modification is used [Proc. Natl. Acad. Sci., 81, 5662-5666 (1984)], or the like method, for instance, can be employed in the conventional manner.

The base sequence of the desired gene obtained in the above manner can be determined and identified, for example by the chemical modification method of Maxam and Gilbert [Maxam-Gilbert, Meth. Enzym., 65, 499-560 (1980)] or the dideoxynucleotide chain termination method using M13 phage [Messing, J. and Vieira, J., Gene, 19, 269-276 (1982)]. Any other methods well known in the art can be employed for that purpose as well without limitation to the methods mentioned above.

Thus there is provided the gene coding for a polypeptide having the specific amino acid sequence mentioned above (such gene is hereinafter referred to as the "desired gene").

Said specific polypeptide can be produced using the above-mentioned desired gene and using a conventional recombinant DNA technology, more specifically, by constructing a recombinant DNA such that the above-mentioned desired gene can be expressed in host cells, introducing the same into the host cells for transformation, and cultivating the resultant transformant.

Eukaryotic cells as well as prokaryotic cells can be used as the host cells. Said eukaryotic cells include vertebrate cells and yeast cells, among others. Frequently used as the vertebrate cells are, for instance, Cos cells [Y. Gluzman, Cell, 23, 175-182 (1981)], which are monkey cells, and a Chinese hamster ovary cell-derived dihydrofolate reductase-deficient cell line [G. Urlaub and L. A. Chasin, Proc. Natl. Acad. Sci. USA, 77, 4216-4220 (1980)], without limitation thereto. An expression vector for use in vertebrate cells generally has a promoter located upstream from the gene to be expressed, an RNA splicing site(s), a polyadenylation site and a transcription termination sequence, among others. It may further have an origin of replication, if necessary. As an example of said expression vector, there may be mentioned pSV2dhfr [S. Subramani, R. Mulligan and P. Berg, Mol. Cell. Biol., 1 (9), 854-864], which contains the early promoter of SV40, for instance, without limitation thereto.

Yeasts are generally used as preferred eukaryotes. Among them, yeasts of the genus Saccharomyces can be used advantageously. Preferably usable as an expression vector in eukaryotic microorganisms such as said yeasts is, for example, pAM82 [A. Miyanohara et al., Proc. Natl. Acad. Sci. USA, 80, 1-5 (1983)], which has a promoter for the acid phosphatase gene.

Escherichia coli and Bacillus subtilis are generally used as preferred prokaryotic hosts, and use can be made, for instance, of an expression plasmid constructed by inserting the desired gene into a plasmid vector replicable in said host bacteria and carrying, upstream from said gene, a promoter and an SD (Shine and Dalgarno) sequence and further ATG necessary for the initiation of protein synthesis so that said desired gene can be expressed. Escherichia coli K12 and the like are often used as Escherichia coli strains for host, while pBR322 is generally used as said vector, without limitation thereto. Various known bacterial strains and vectors can be used as well. Usable as the promoter are, for example, the tryprophan promoter, $P_L$ promoter, lac promoter and lpp promoter; in each case, the desired gene can be expressed.

In an example where the tryptophan promoter is used, detailed description may be made as follows: A tryptophan promoter- and SD sequence-containing vector, pTM1 [Fumio Imamoto: Taisha (Metabolism and Disease), vol. 22, 289 (1985)] is used as the expression vector and a gene coding for a desired polypeptide as provided, if necessary, with ATG is ligated therewith at the restriction enzyme ClaI site downstream from the SD sequence.

Not only a direct expression system but also a fused protein expression system in which $\beta$-galactosidase or $\beta$-lactamase is utilized can be employed.

Employable as the method of introducing the thus-obtained expression vector into host cells for transformation of said cells are general methods, for example the method comprising collecting cells predominantly in the logarithmic growth phase, treating them with $CaCl_2$ to facilitate spontaneous uptake of DNA and then causing uptake of the vector. In the method mentioned above, it is also possible to use $MgCl_2$ or RbCl simultaneously in the culture medium for further improvement of transformation, as generally known in the art. The method comprising converting host cells to spheroplasts or protoplasts and then subjecting these to transformation is also employable.

The thus-obtained desired transformant can be cultivated by a conventional method, whereby the desired polypeptide is produced and accumulated. The medium to be used for said cultivation may be any of various media conventionally used for ordinary cell culture, typical examples of which are, for example, L medium, E medium and M9 medium, and media wherein various conventional carbon sources, nitrogen sources, inorganic salts, vitamins, etc. are added to these media. In cases where the above-mentioned tryptophan promoter is used, it is generally possible to perform cultivation using, for example, M9 minimal medium supplemented with casamino acids for causing the promoter to function. It is also possible to add an agent for increasing the functioning of the tryptophan promoter, for example indoleacrylic acid, to said medium at an appropriate time point(s) during cultivation.

The desired polypeptide, namely the above-mentioned specific polypeptide, can be purified and isolated from the thus-obtained active substance-containing culture mass by a conventional method. In extracting said polypeptide from the host, it is more preferable, from the higher structure retention viewpoint, to employ mild conditions, for example the osmotic pressure shock method.

The purification and isolation mentioned above can be accomplished using various treatment procedures in which physical and chemical properties, among others, of the polypeptide in question are utilized [see, for example, "Seikagaku (Biochemistry) Data Book II", pp. 1175-1259, 1st edition, 1st printing, published June 23, 1980 by Tokyo Kagaku Dojin]. Specifically, the following methods, for instance, can be employed: treatment with a conventional protein precipitating agent, ultrafiltration, molecular sieve chromatography (gel filtration), liquid chromatography, centrifugation, electrophoresis, affinity chromatography, dialysis, and combinations of these.

More specifically, the above procedure can be carried out, for example, in the following manner. Thus, the desired polypeptide is first partially purified in advance from the culture supernatant. This partial purification can be effected, for example, by treatment using, as a protein precipitating agent, an organic solvent, such as acetone, methanol, ethanol, propanol or dimethylformamide (DMF), or an acid, such as acetic acid, perchloric acid (PCA) or trichloroacetic acid (TCA), treatment with a salting-out agent, such as ammonium sulfate, sodium sulfate or sodium phosphate, and/or ultrafiltration treatment using a dialyzer membrane, a flat membrane or a hollow fiber membrane. The mode of operation and conditions of each of these treatment may be the same as those conventionally employed.

The roughly purified product obtained in the above manner is then subjected to gel filtration and fractions showing an activity of the desired substance are collected. Gel filtration media usable for this purpose include, but are not particularly limited to, those made of such materials as dextran gel, polyacrylamide gel, agarose gel, polyacrylamide-agarose gel and cellulose. As specific examples of these, there may be mentioned Sephadex G type, Sephadex LH type, Sepharose type, Sephacryl type (all available from Pharmacia), Cellofine (Chisso), Biogel P type, Biogel A type (Bio-Rad), Ultrogel (LKB) and TSK-G type (Tosoh), among others.

The desired polypeptide can be further purified and isolated as a homogeneous substance by subjecting the active fractions obtained by the above gel filtration to, for example, affinity chromatography using a hydroxyapatite column, ion exchange column chromatography by the DEAE, CM, SP or the like method, chromatofocusing or reversed-phase high performance liquid chromatography or to a combination of these techniques.

The above-mentioned chromatofocusing can be carried out by various known methods. PBE94 (Pharmacia), for instance, can be used as the column, imidazole-hydrochloric acid, for instance, can be used as the initial buffer, and Polybuffer 74 (Pharmacia)-hydrochloric acid (pH 4.0), for instance, can be used as the eluent.

The above-mentioned reversed-phase high performance liquid chromatography can be conducted using, for example, a $C_4$ Hipore reversed-phase HPLC column (Bio-Rad Laboratories), with acetonitrile, trifluoroacetic acid (TFA), water, or the like, or a mixed solvent composed of these as the mobile phase.

IL-1$\beta$ and the above-specified polypeptides as derivatives of IL-1$\beta$ can thus be isolated and recovered.

The composition according to the invention contains human serum albumin and a saccharide together with an IL-1$\beta$ active substance, such as the above-mentioned IL-1$\beta$ or a derivative thereof.

The composition according to the invention may contain a saccharide, a surfactant and human serum albumin together with an IL-1$\beta$ active substance, such as the above-mentioned IL-1$\beta$ or a derivative thereof.

The saccharide mentioned above is not limited to any particular species. Thus, for example, monosaccharides, such as glucose, mannose, galactose and fructose, sugar alcohols, such as mannitol, inositol and xylitol, disaccharides, such as sucrose, maltose and lactose, and polysaccharides, such as dextran and hydroxypropyl-starch, can be used either alone or as a mixture of two or more of them. Among them, sucrose, maltose, mannitol, inositol and dextran, for instance are particularly preferred.

The surfactant is not limited to any particular species, either. Thus all ionic and nonionic surfactants can be used. Among them, such surfactants as polyoxyethylene glycol sorbitan alkyl esters, polyoxyethylene alkyl ethers, sorbitan monoacyl esters and fatty acid glycerides can preferably be used.

The addition level of the above-mentioned saccharide is suitably not less than about 0.1 mg, preferably within the range of about 1 to 100 mg, per microgram ($\mu$g) of IL-1$\beta$ active substance, while the addition level of the surfactant is suitably not less than about 0.0001 mg, preferably within the range of about 0.001 to 0.1 mg, per microgram of IL-1$\beta$ active substance. The addition level of human serum albumin is suitably not less than about 0.001 mg, preferably within the range of about 0.01 to 10 mg, per microgram of IL-1$\beta$ active substance.

The composition according to the invention contains an interleukin-1$\beta$ active substance, human serum albumin and saccharide as essentials but, otherwise, may contain similar to ordinary pharmaceutical compositions of this kind. Thus it may optionally contain another or other pharmacologically active ingredients and/or one or more ingredients commonly used in the pharmaceutical practice. The thus-obtained compositions can be effectively applied to the above-mentioned various pharmaceutical uses, for example, as immunostimulants for enhancing antibody production or potentiating vaccines or for the treatment of immunodeficiency, antitumor agents, cytokine production promoters, antiinflammatory agents, radiation damage preventing agents, therapeutic agents for opportunistic infection, and so forth.

In a preferred embodiment of the invention, a sulfur-containing reducing agent is added to the composition according to the invention so that the stability of the IL-1$\beta$ active substance can be further increased.

Said sulfur-containing reducing agent may be an ordinary one and preferred examples thereof include, among others, cysteine, N-acetylhomocysteine, thioctic acid, thioglycolic acid and salts of these and, further, such relatively mild reducing agents as thioethanolamine, thioglycerol, sodium thiosulfate, thiolactic acid, dithiothreitol and glutathione. These may be used singly or two or more of them may be used combinedly. The addition level of these is not particularly critical but is suitably not less than about 0.001 mg, preferably about 0.01 to 10 mg (as the total when two or more are used combinedly), per microgram of IL-1$\beta$ active substance.

In a further preferred embodiment of the invention, the above-mentioned composition according to the invention is isotonized with a buffer solution to give an isotonized pharmaceutical preparation.

As typical examples of the buffer solution to be used for that purpose, there may be mentioned various buffer solutions with a pH of about 4 to 8, preferably 5 to 6, such as citric acid-sodium citrate, citric acid-sodium phosphate, acetic acid-sodium acetate and citric acid-borax buffers.

The composition according to the invention is prepared in the form of a pharmaceutical composition, for example by compounding an IL-1$\beta$ active substance generally in a pharmacologically effective amount and the above-mentioned specific ingredient(s) together with a pharmaceutically appropriate carrier(s). Those pharmaceutical carries that are generally used in producing pharmaceutical preparations depending on the dosage form are all usable as said carriers for pharmaceutical preparation production. Thus usable are such excipients or diluents as filters, extenders, binders, humectants and disintegrants. The form of the pharmaceutical composition is not particularly limited so far as it contains an IL-1$\beta$ active substance, for example, solid forms such as tablets, powders, granules and pills and injectable liquid forms such as solutions, suspensions and emulsions. Furthermore, said composition can be in the form of a dried product, which can be rendered liquid by addition of an appropriate carrier prior to use. These pharmaceutical compositions can be prepared by a conventional method.

The pharmaceutical preparation thus obtained can be administered via an appropriate route selected depending on the form of said pharmaceutical composition. When it is in the form of an injection, for instance, the pharmaceutical preparation can be administered intravenously, intramuscularly, subcutaneously, intradermally or intraperitoneally, for instance. When in solid form, the pharmaceutical composition can be administered orally or enterally. The content of the active ingredient in the pharmaceutical preparation and the dose of said preparation may vary depending on the method of administration of said preparation, the mode of administration, the purposes of use, the symptom of the patient to whom the preparation is applied, and other factors. Generally, however, it is desirable that a preparation form containing the active ingredient in an amount of about 0.0000001 to 80% by weight be prepared and that this preparation be administered in a daily dose, calculated as the active ingredient contained therein, of about 0.001 to 100 $\mu$g per human adult. Said daily dose needs not to be given as a single dose but may be administered in three or four divided doses.

In accordance with the invention, a composition is provided in which the IL-1$\beta$ active substance, which has attracted attention and is expected to serve as a drug, is stabilized.

The composition according to the invention has an outstanding feature with respect to the stability of the constituent IL-1$\beta$ active substance and can remain stable during processing for the production of desired ordinary pharmaceutical forms, such as freezing or lyophilization treatment, and further for a prolonged period of time under ordinary storage conditions for the pharmaceutical products. The composition is very useful in such field.

BRIEF DESCRIPTION OF THE DRAWINGS

Fig. 1 to Fig. 3 graphically represent the results of testing of Polypeptide I for CSF production promoting effect.

Fig. 4 graphically shows the results of testing of Polypeptide I for antiarthritic activity.

Fig. 5 graphically shows the results of testing of several IL-1$\beta$ derivatives for CSF production promoting activity.

Fig. 6 graphically shows the results of testing of a certain IL-1$\beta$ derivative for antiinflammatory activity.

Fig. 7 graphically shows the results of testing of a certain IL-1$\beta$ derivative for radiation damage preventing activity.

Fig. 8 graphically shows the results of testing of a certain IL-1$\beta$ derivative for opportunistic infection preventing activity.

EXAMPLES

The following examples are further illustrative of the present invention.

The IL-1$\beta$ active substances (IL-1$\beta$ derivatives) used as active ingredients in each example are represented by the symbols defined below in Table 1. Among them, the polypeptides I to XXXXVIII are described in Japanese Kokai Tokkyo Koho No. 63-152398. The polypeptides XXXXIX to XXXXXVI are obtainable by the same method as disclosed in the above-cited reference, as described hereinafter in reference production examples. For measuring the IL-1 activity and GIF activity of these, the methods described in the above-cited reference (Kokai Tokkyo Koho) are used.

Table 1

| Symbol | IL-1β derivative |
|--------|------------------|
| I | IL-1β itself |
| II | [Gly⁴]IL-1β (IL-1β with 4-position Arg replaced by Gly) |
| III | [Gln¹¹]IL-1β (IL-1β with 11-position Arg replaced by Gln) |
| IV | [Ser⁸]IL-1β (IL-1β with 8-position Cys replaced by Ser) |
| V | [Ser⁸ Ser⁷¹]IL-1β (IL-1β with 8-position Cys and 71-position Cys replaced by 8-position Ser and 71-position Ser, respectively) |
| VI | [Ser⁷¹]IL-1β (IL-1β with 71-position Cys replaced by Ser) |
| VII | [Gln¹⁰³]IL-1β (IL-1β with 103-position Lys replaced by Gln) |
| VIII | IL-1β(1-140) polypeptide (IL-1β with 141-position and subsequent positions deleted) |
| IX | [Gln¹²¹]IL-1β (IL-1β with 121-position Tyr replaced by Gln) |
| X | Met-IL-1β (IL-1β with Met attached to N terminus) |
| XI | des-Ala¹-IL-1β (IL-1β with 1-position Ala deleted) |
| XII | [Leu⁹⁸]IL-1β (IL-1β with 98-position Arg replaced by Leu) |
| XIII | [Gly⁴Gln¹¹Leu⁹⁸]IL-1β (IL-1β with 4-position Arg, 11-position Arg and 98-position Arg replaced by 4-position Gly, 11-position Gln and 98-position Leu, respectively) |
| XIV | [Gly⁴Gln¹¹]IL-1β (IL-1β with 4-position Arg and 11-position Arg replaced by 4-position Gly and 11-position Gln, respectively) |
| XV | [Gly⁴Leu⁹⁸]IL-1β (IL-1β with 4-position Arg and 98-position Arg replaced by 4-position Gly and 98-position Leu, respectively) |
| XVI | [Gln¹¹Leu⁹⁸]IL-1β (IL-1β with 11-position Arg and 98-position Arg replaced by 11-position Gln and 98-position Leu, respectively) |
| XVII | des-Arg⁴-IL-1β (IL-1β with 4-position Arg deleted) |
| XVIII | des-Arg¹¹-IL-1β (IL-1β with 11-position Arg deleted) |
| XIX | des-Arg⁹⁸-1L-1β (IL-1β with 98-position Arg deleted) |
| XX | [Ala⁸]IL-1β (IL-1β with 8-position Cys replaced by Ala) |
| XXI | des-Cys⁸-IL-1β (IL-1β with 8-position Cys deleted) |
| XXII | [Ala⁷¹]IL-1β (IL-1β with 71-position Cys replaced by Ala) |
| XXIII | [Val⁷¹]IL-1β (IL-1β with 71-position Cys replaced by Val) |
| XXIV | des-Cys⁷¹-IL-1β (IL-1β with 71-position Cys deleted) |
| XXV | [Leu⁹³]IL-1β (IL-1β with 93-position Lys replaced by Leu) |
| XXVI | IL-1β-(1-144) polypeptide (IL-1β with amino acid residues at 145-position and subsequent positions deleted) |
| XXVII | IL-1β-(1-148) polypeptide (IL-1β with amino acid residues at 149-position and subsequent positions deleted) |
| XXVIII | [Arg¹²⁰]IL-1β (IL-1β with 120-position Trp replaced by Arg) |
| XXIX | [Tyr³⁰]IL-1β (IL-1β with 30-position His replaced by Tyr) |
| XXX | IL-1β-(1-102) polypeptide (IL-1β with amino acids at 103-position and subsequent positions deleted) |
| XXXI | (77'-116')-IL-1β (IL-1β with amino acid sequence covering 77'-116' positions of formula (B) attached to N terminus) |
| XXXII | (71'-116')-IL-1β (IL-1β with amino acid sequence covering 71'-116' positions of formula (B) attached to N terminus) |

| Symbol | IL-1β derivative |
|---|---|
| XXXIII | (32'-116')-IL-1β (IL-1β with amino acid sequence covering 32'-116' positions of formula (B) attached to N terminus) |
| XXXIV | (1'-116')-IL-1β (IL-1β with amino acid sequence covering 1'-116' positions of formula (B) attached to N terminus) |
| XXXV | IL-1β-(1-102) polypeptide (IL-1β with 121-position and subsequent positions deleted) |
| XXXVI | $[Ala^8Ser^{71}]$IL-1β (IL-1β with 8-position Cys and 71-position Cys replaced by 8-position Ala and 71-position Ser, respectively) |
| XXXVII | $[Ala^8Ala^{71}]$IL-1β (IL-1β with 8-position Cys and 71-position Cys replaced by 8-position Ala and 71-position Ala, respectively) |
| XXXVIII | $[Ala^8Val^{71}]$IL-1β (IL-1β with 8-position Cys and 71-position Cys replaced by 8-position Ala and 71-position Val, respectively) |
| XXXIX | IL-1β-(4-153) polypeptide (IL-1β with amino acid sequence covering 1-position Ala to 3-position Val deleted) |
| XXXX | IL-1β-(7-153) polypeptide (IL-1β with amino acid sequence covering 1-position Ala to 6-position Leu deleted) |
| XXXXI | IL-1β-(10-153) polypeptide (IL-1β with amino acid sequence covering 1-position Ala to 9-position Thr deleted) |
| XXXXII | des-$Val^3$-IL-1β (IL-1β with 3-position Val deleted) |
| XXXXIII | des-$Ser^5$-IL-1β (IL-1β with 5-position Ser deleted) |
| XXXXIV | des-$Lys^{97}$-IL-1β (IL-1β with 97-position Lys deleted) |
| XXXXV | des-$Phe^{99}$IL-1β (IL-1β with 99-position Phe deleted) |
| XXXXVI | IL-1β-(1-150) polypeptide (IL-1β with amino acid residues at 151-position and subsequent positions deleted) |
| XXXXVII | des-$Ser^{153}$-IL-1β (IL-1β with 153-position Ser deleted) |
| XXXXVIII | Met-$[Gly^4]$IL-1β (IL-1β with Met at N terminus and with 4-position Arg replaced by Gly) |
| XXXXIX | $[Lys^4]$IL-1β (IL-1β with 4-position Arg replaced by Lys) |
| XXXXX | $[Gln^4]$IL-1β (IL-1β with 4-position Arg replaced by Gln) |
| XXXXXI | $[Asp^4]$IL-1β (IL-1β with 4-position Arg replaced by Asp) |
| XXXXXII | $[Asp^{93}]$IL-1β (IL-1β with 93-position Lys replaced by Asp) |
| XXXXXIII | (112'-116')-IL-1β (IL-1β with amino acid sequence covering 112'-116' positions of formula (B) added to N terminus) |
| XXXXXIV | Asp-IL-1β (IL-1β with Asp added to N terminus) |
| XXXXXV | Leu-IL-1β (IL-1β with Leu added to N terminus) |
| XXXXXVI | Arg-IL-1β (IL-1β with Arg added to N terminus) |

Reference Production Example 1

(1) The polypeptides (IL-1β derivatives) shown below in Table 2 were obtained through site-specific mutagenesis using the plasmid ptrpGIF-α, as described in Japanese Kokai Tokkyo Koho No. 63-152398 in Production Example 1-(1) thereof.

The expression of each polypeptide, GIF activity measurement and purification were performed as described in the above publication (Kokai Tokkyo Koho). SDS-PAGE was also performed as described in said reference in Reference Example 2-(4) thereof. Unless otherwise specified, the same applies in the subsequent examples.

Table 2

| Polypeptide | Primer | |
|---|---|---|
| XXXXIX | 5'-CTCCTGTAAAATCTCTGA-3' | |
| XXXXX | 5'-CTCCTGTACAATCTCTGA-3' | |
| XXXXXI | 5'-CTCCTGTAGATTCTCTGA-3' | |
| XXXXXII | 5'-ACCCAAAGGATAAGATGG-3' | |
| Polypeptide | Plasmid constructed | Host (E. coli) |
| XXXXIX | ptrpGIF-$\alpha$-4K | HB101 |
| XXXXX | ptrpGIF-$\alpha$-AQ | HB101 |
| XXXXXI | ptrpGIF-$\alpha$-4D | HB101 |
| XXXXXII | ptrpGIF-$\alpha$-93D- | HB101 |
| Polypeptide | GIF activity (units/ml culture fluid) | M. W. (kd) |
| XXXXIX | $7.4 \times 10^5$ | 17.5 |
| XXXXX | $2.1 \times 10^5$ | 17.5 |
| XXXXXI | $1.1 \times 10^3$ | 17.5 |
| XXXXXII | $3.5 \times 10^3$ | 17.5 |

Reference Production Example 2

In this example, IL-1$\beta$ derivatives (polypeptides XXXXXIII to XXXXXVI) with various amino acid residues attached to the N terminus of IL-1$\beta$ were produced.

In the vicinity of the above-mentioned N-terminal region, there is a ClaI site 5 bases upstream from the protein synthesis initiation codon ATG. Downstream therefrom, however, there is only an MspI site at the 11th amino acid Arg. For insertion of a linker DNA between these two sites, said linker will become too long. Therefore, attempts were made to create an appropriate restriction enzyme site between these restriction enzyme sites in an amino acid region closer to the N terminus. As a result, the codon CGT for the fourth Arg was replaced by AGA to form, together with the next Ser, a BglII restriction enzyme site, AGATCT. Various synthetic linkers synthesized in advance and containing a mutation region were each inserted between the BglII site thus produced and the ClaI site mentioned above. The thus-constructed desired genes were allowed to be expressed in Escherichia coli, whereby the desired polypeptides were obtained. This process is detailedly described in the following.

(1) Construction of IL-1$\beta$ expression plasmid having restriction enzyme BglII site

ptrpGIF-$\alpha$ with trp promoter used therein (4921 bp) was digested with the restriction enzymes ClaI and BamHI and two fragment DNAs (579 bp and 4342 bp) were obtained.

The above-mentioned 579 bp fragment DNA was further digested with MspI to give a 543 bp fragment DNA having an MspI site at the 5' end and a BamHI site at the 3' end.

A linker DNA having the sequence shown below was then synthesized and phosphorylated at the 5' end using T4 polynucleotide kinase.

C l a I                                                B g l II

5'- C G A T A A T G G C T C C T G T A A G A

3'-      T A T T A C C G A G G A C A T T C T

M s p I

T C T C T G A A C T G C A C T C T      -3'

A G A G A C T T G A C G T G A G A G G C -5'

This phosphorylated linker and the above-mentioned 543 bp fragment were ligated together using T4 DNA ligase. Thus there was obtained a DNA fragment (579 bp) having a ClaI site at the 5' end, a BglII site and an MspI site in the middle, and a BamHI site at the 3' end.

Finally, the 579 bp fragment obtained as described above was ligated with the 4342 bp fragment mentioned previously using TA DNA ligase. Thus there was obtained an IL-1$\beta$ expression plasmid (ptrpIL-1$\beta$,H2; 4921 bp) having a newly-formed BglII site.

The thus-obtained plasmid was used to transform Escherichia coli HB101 and clones obtained were subjected to DNA sequencing. As a result, it was confirmed that the plasmid contained an IL-1$\beta$ expression gene having a BglII site, as desired.

(2) Production of IL-1$\beta$ mutant proteins with N-terminal addition using synthetic DNA linkers

Mutant proteins with a desired amino acid attached to the N terminus of IL-1$\beta$ were produced by substitution, of a synthetic DNA linker for the vector DNA, between the ClaI site and BglII site of the plasmid ptrpIL-1$\beta$,H2.

Selected as the amino acid to be added were an IL-1$\beta$ precursor-derived 5-amino-acid sequence (Ala-Tyr-Val-His-Asp; AYVHD), an acidic amino acid (Asp; D), a neutral amino acid (Leu; L) and a basic amino acid (Arg; R). The corresponding mutant proteins are represented by the abbreviations [AYVHD]-IL-1$\beta$, [D]-IL-1$\beta$, [L]-IL-1$\beta$ and [R]-IL-1$\beta$, respectively.

The synthetic linker DNA sequence are as follows:

(1) Linker for attachment of AYVHD

```
              C la I
                 |
                 |
                 |                      Ala   Tyr   Val   His
                 |                         |
        5'-| C G A T A A T G G C|A T A T G T G C A C
        3'-     | T A T T A C C G T A|T A C A C G T G
                 |                     |
                                     N de I


        Asp   Ala   Pro   Val        Bgl II
                                        |
        G A T G C T C C T G T A A |          -3'
        C T A C G A G G A C A T T C T A G |  -5'
                                        |
                                        |
```

(2) Linker for attachment of D

```
              C la I
                 |
                 |
                 |                 Asp   Ala   Pro   Val
                 |
        5'-| C G A T A A T G G A T G C T C C T G T A
        3'-     | T A T T A C C T A C G A G G A C A T
                 |


        Bgl II
          |
        A |          -3'
        T C T A G |  -5'
          |
```

(3) Linker for attachment of L

```
C la I
  |
  |                          Leu  Ala  Pro  Val
  |
5'-| C G A T A A T G T T A G C T C C T G T A
3'-    | T A T T A C A A T C G A G G A C A T
       |


   B gl II
     |
A    |                -3'
     |
T C T A G    |-5'
             |
```

(4) Linker for attachment of R

```
C la I
  |
  |                          Arg  Ala  Pro  Val
  |
5'-| C G A T A A T G C G T G C T C C T G T A
3'-    | T A T T A C G C A C G A G G A C A T
       |


   B gl II
     |
A    |                -3'
     |
T C T A G    |  -5'
             |
```

First, the IL-1β expression plasmid ptrpIL-1β,H2 was digested with ClaI and BgIII and a 4.9 kbp fragment DNA was recovered.

Then, the above-mentioned linkers (1) to (4) were synthesized and phosphorylated at the 5' end using T4 polynucleotide kinase.

The thus-obtained 5'-phosphorylated synthetic linkers were respectively ligated to the above-mentioned 4.9 kbp fragment DNA using T4 DNA ligase. Thus there were constructed four expression plasmids for the respective mutant proteins with N-terminal attachment.

These plasmids were respectively used to transform Escherichia coli HB101 and clones obtained were subjected to DNA sequencing for confirming the desired DNA sequence.

(3) Expression and purification of IL-1β-derived mutant proteins with N-terminal addition

The strain HB101 (K12 strain) transformed with each of the expression plasmids obtained as described above in (2) was shake-cultured in M9 medium at 37°C.

Cells harvested by centrifugation were treated with 1 M disodium hydrogen phosphate at 4°C, followed by dialysis against 5 mM Tris-hydrochloric acid buffer (pH 8.0), whereby cells were disrupted by osmotic pressure shock.

16

The dialyzate was adjusted to a pH of about 4, the resultant precipitate was removed by centrifugation and the supernatant was subjected to anion exchange high performance liquid chromatography (HPLC; Tosoh model SP-5PW column). The main peak was subjected to rechromatography for further purification. A total of two repetitions of the above-mentioned anion exchange HPLC resulted in each IL-1$\beta$ mutant protein purified to an extent such that it gave almost a single band in SDS-PAGE electrophoresis.

Furthermore, ultrafiltration with an Amicon YM-5 membrane was conducted for substitution of water for the solvent acetate buffer (pH 5.5).

Some properties of the mutant proteins obtained are summarized below in Table 3.

Table 3

| Mutant protein | Molecular weight (kd) | Isoelectric point |
|---|---|---|
| [AYVHD]-IL-1$\beta$ | 18.5 | 6.4 |
| [D]-IL-1$\beta$ | 18 | 5.9 |
| [L]-IL-1$\beta$ | 18 | 6.7 |
| [R]-IL-1$\beta$ | 18 | 7.2 |
| IL-1$\beta$ | 18 | 6.9 |

The IL-1$\beta$ (polypeptide I) and derivatives thereof were subjected to the following activity testing.

The LAF activity data (U, per mg of protein calculated as polypeptide I) as measured by RIA using the above-mentioned antiserum to polypeptide I were as shown below in Table 4.

Table 4

| Polypeptide | LAF activity (U/mg) |
|---|---|
| Polypeptide I | $4.5 \times 10^6$ |
| Polypeptide II | $1.6 \times 10^5$ |
| Polypeptide III | $6.2 \times 10^6$ |
| Polypeptide IV | $2.6 \times 10^5$ |
| Polypeptide V | $2.1 \times 10^5$ |
| Polypeptide VI | $1.3 \times 10^7$ |
| Polypeptide VIII | $8.0 \times 10^3$ |

Pharmacological Test Example 1:

Testing of polypeptide I for CSF production promoting effect

(1) Test for promotion effect on CSF production by human pulmonary cells

The following test was performed using the human pulmonary cell-derived cell line HFL-1 (human embryonic lung fibroblasts; ATCC accession number CCL-153) as a CSF-producing cell line.

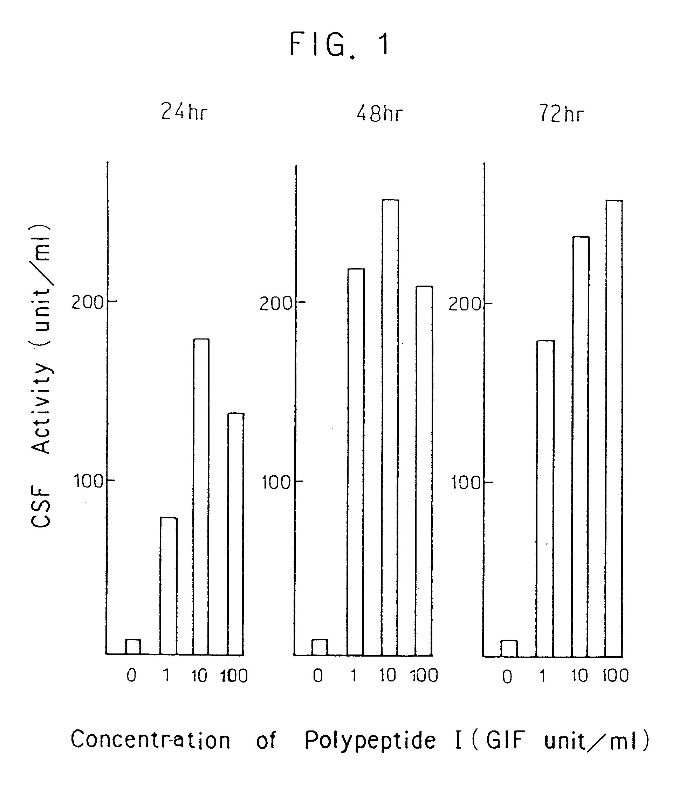

First, the above HFL-1 cells were suspended in Ham 12K medium [Ham, R. G., Proc. Natl. Acad. Sci., 53, 288 (1965)] supplemented with 10% fetal calf serum to a cell concentration of $2 \times 10^5$ cells/ml. Then, the polypeptide I obtained above in Reference Example 1 was added, in various concentrations, to the above-mentioned cell suspension, and incubation was conducted at 37°C in a carbon dioxide gas incubator for 24, 48 or 72 hours. Each culture supernatant was collected and the amount of CSF produced and accumulated in the culture supernatant was measured using mouse marrow cells [Lewis, I. C. et al., J. Immunol., 128, 168 (1982)]. The results obtained with the polypeptide I after the respective incubation periods (hrs) are shown in Fig. 1. In the figure, the abscissa is for the concentration of polypeptide I (GIF units/ml) and the ordinate for the CSF activity (units/ml).

From the above results, it is evident that the addition of polypeptide I enhances the production of CSF by the cell line HFL-1 by a factor as high as several hundred as compared with the case where the addition of said polypeptide is omitted.

17

(2) Testing of polypeptide I for CSF production effect in human skin-derived cells

The following test was performed using CRL-1445 (ATCC No.) as a human normal skin-derived cell line. Thus, the above cells were suspended in Dulbecco's MEM medium [Dulbecco, R. and Freeman, G., Virology, 8, 396 (1959)] supplemented with 10% fetal calf serum to a concentration of $2 \times 10^5$ cells/ml. The polypeptide I obtained in the reference example was added, in various concentrations, to the above cell suspension, and incubation was performed at 37°C in a carbon dioxide gas incubator for 24, 48 or 72 hours. Each culture supernatant was collected and the amount of CSF produced was determined in the same manner as in the above-mentioned test (1) using mouse marrow cells.

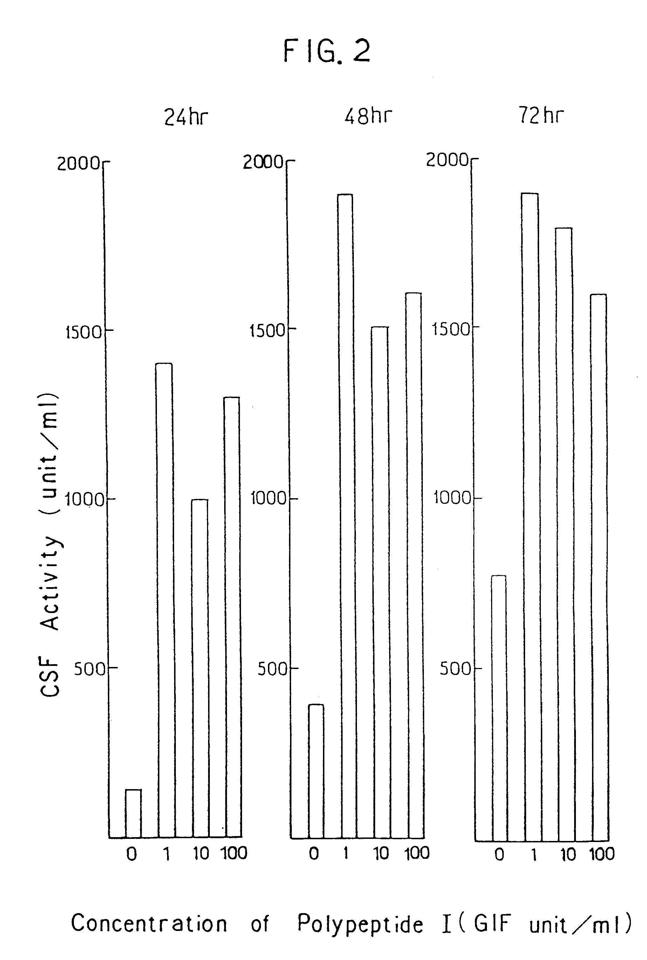

The results obtained are shown in Fig. 2 in the same manner as in Fig. 1.

From Fig. 2, it is evident that the ability of human normal skin-derived protofibroblasts to produce CSF is markedly enhanced by the addition of polypeptide I to said cells in an amount of not less than 1 unit/ml as expressed in terms of GIF activity.

(3) Test for CSF production promoting effect in vivo

The following animal test was performed to confirm the fact that when the polypeptide I is administered to living organisms, a CSF production enhancing effect is produced in vivo.

Thus, the polypeptide I obtained in the reference example was intravenously administered to normal mice (BALB/C mice, purchased from Shizuoka Laboratory Animal Center) in various doses ($10^3$ to $10^5$ units per individual as expressed in terms of GIF activity). At 2, 4, 8, 12 and 24 hours after the above administration, blood samples were collected from each experimental animal and the sera were assayed for CSF concentration using mouse marrow cells.

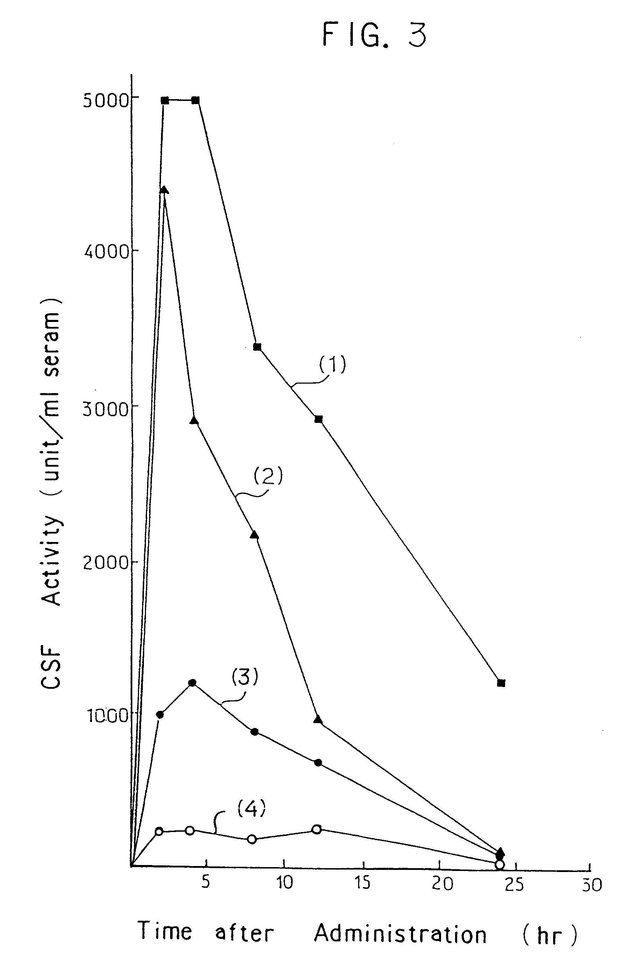

The results are shown in Fig. 3. In the figure, the abscissa is for the time (hrs) after administration of various concentrations (GIF units/individual) of polypeptide I, and the ordinate for the CSF activity (units/ml of serum). In the figure, (1) stands for the group given 100000 GIF units/individual of polypeptide I, (2) for the 10000 GIF units/individual group, (3) for the 1000 GIF units/individual group, and (4) for a control group (HSA 10 $\mu$g/individual group).

Fig. 3 revealed that when the polypeptide I was administered to the animals, the CSF concentration in the animal serum markedly increased. Thus it was noted that the polypeptide I is capable of significantly increasing the in vivo CSF production in proportion to the quantity injected.

Pharmacological Test Example 2:

Testing of polypeptide I for antiarthritic activity

(1) Rats with adjuvant arthritis were prepared by the method of Pearson [Pearson, C. M., Proc. Soc. Exp. Biol. Med., 91, 95 (1956)] and of Ward and Jones [Ward, J. R., Jones, R. S., Arthritis Rheumatism, 5, 557 (1962)]. Thus, an adjuvant as prepared by suspending dead cells of Mycobacterium butyricum in liquid paraffin was injected into S. D. strain female rats intradermally at the tail base in a dose of 0.05 ml. On day 14, the animals were grouped (n = 6) according to the extent of paw swelling. For a period of 5 days (days 15 to 19), the polypeptide I or the solvent therefor (physiological saline: control group) was intradermally administered and the effect on arthritis was evaluated by measuring the paw volume at regular intervals.

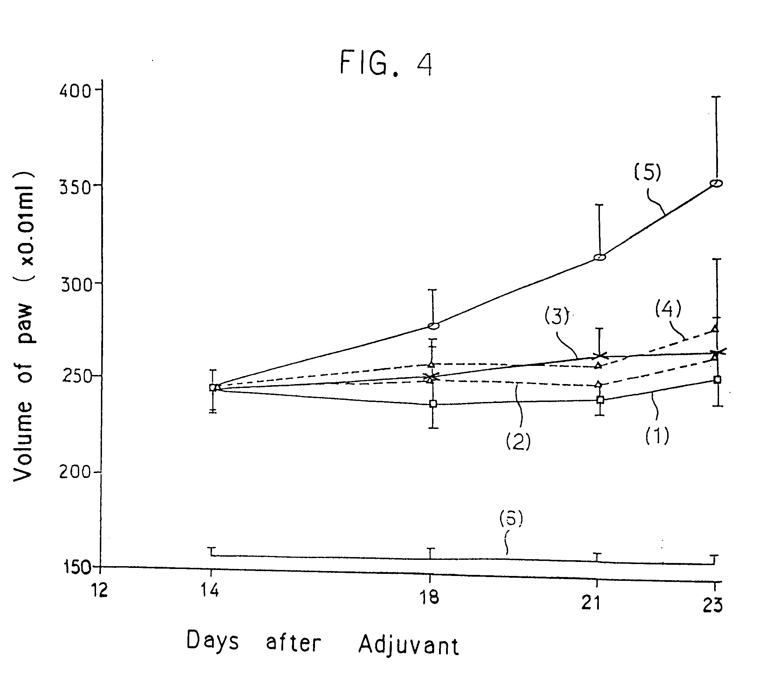

The results are shown in Fig. 4. In the figure, the abscissa is for the number of days following adjuvant administration while the ordinate is for the paw volume (x 0.01 ml). In the figure, (1) stands for the group given 100000 GIF units/individual of polypeptide I, (2) for the 10000 GIF units/individual group, (3) for the 1000 GIF units/individual group, (4) for the 100 GIF units/individual group, (5) for the control group (physiological saline group), and (6) for the normal rat group.

As seen in Fig. 4, the paw swelling in the control group [graph (5)] became worsened until day 23 whereas, in the polypeptide I groups [graphs (1) to (4), a paw swelling suppressing effect was observed from the 4th day (day 18 following adjuvant administration). It was also confirmed that the progress of arthritis could be prevented even 4 days after the final administration (i.e. day 23 after adjuvant administration).

Pharmacological Test Example 3:

Testing of IL-1$\beta$ derivatives for CSF production promoting effect

The following test was performed using the cell line U-373MG [ATCC HTB17, glioblastoma, astrocytoma, human].

The cells mentioned above were suspended in Eagle's MEM medium (Nissui) supplemented with 10% FCS (GIBCO), MEM non-essential amino acids (Flow) and MEM sodium pyruvate (Flow) to a cell concentration of 2 x $10^5$ cells/ml, the test substance was added in various concentrations, and incubation was performed at 37°C in a carbon dioxide gas incubator for 24 hours.

Each culture supernatant was collected and the amount of CSF produced and accumulated in the culture supernatant was determined using mouse marrow cells [Lewis, I. C. et al., J. Immunol., 128, 168 (1982)].

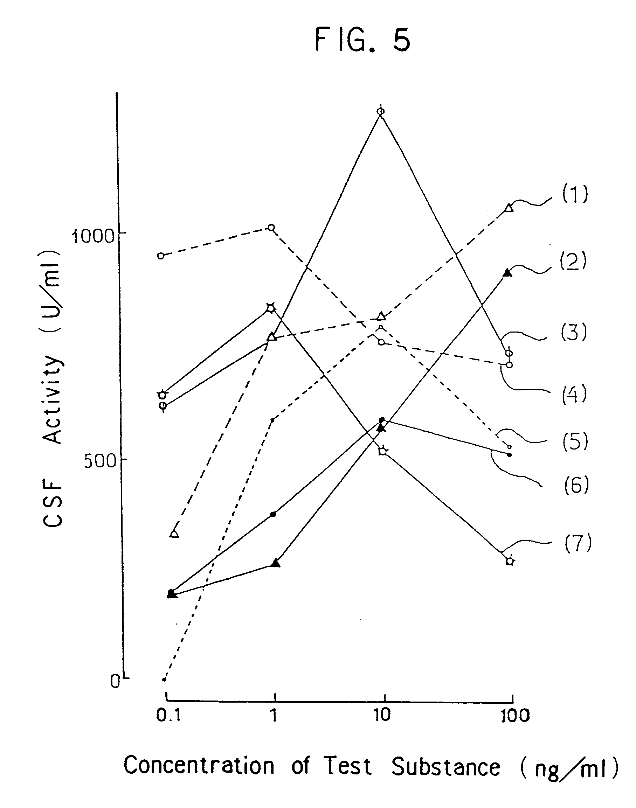

The results are shown in Fig. 5. In the figure, the abscissa is for the concentration (ng/ml) of the test substance and the ordinate for the CSF activity (U/ml). In the figure, the curves (1) to (7) indicate the results obtained using the following polypeptides as test substances:

Curve (1) ... Polypeptide VI,
Curve (2) ... Polypeptide II,
Curve (3) ... Polypeptide VIII,
Curve (4) ... Polypeptide V,
Curve (5) ... Polypeptide IV,
Curve (6) ... Polypeptide III,
Curve (7) ... Polypeptide XXX.

Pharmacological Test Example 4:

Testing of IL-1$\beta$ derivatives for antiinflammatory activity

This test was performed according to the method of Winter et al. [Proc. Soc. Exptl. Biol. Med., 111, 544-547 (1962)].

Thus, on the day before experiment, male rats of 6 to 8 weeks of age (Sprague Dawley strain, Charles River Japan) were divided into groups of 6 to 8 animals based on the body weight. A 1% suspension of carrageenin (Marine Colloid) in physiological saline was used as an inflammation producing agent (inducer) and injected subcutaneously into the right hind paw of the rats at a dose of 0.1 ml to thereby induce paw swelling. For evaluating the paw swelling, the right hind paw volume was measured at specified time points before and after inducer injection using a plethysmometer (Ugo-Vasile). The percent volume increase reltive to the volume before inducer injection was expressed as a swelling percentage (swelling %).

The test substance was dissolved and diluted in Dulbecco's phosphate buffered saline and each solution was injected intradermally into the back of the rat in a dose of 0.1 ml 1 hour before inducer administration. A solvent administration group was prepared as a control group and subjected to the same experiment.

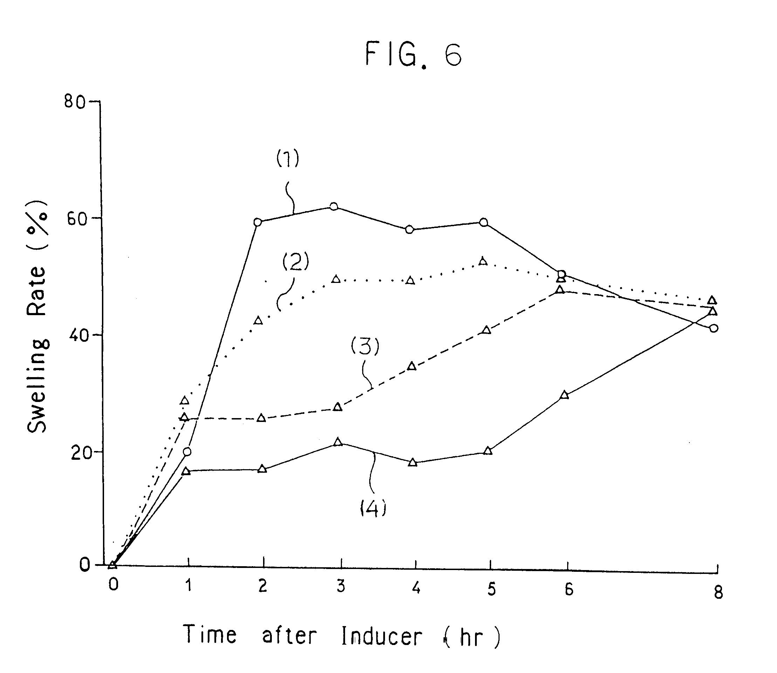

The results are shown in Fig. 6.

In the figure, the abscissa is for the time (hrs) after inducer administration and the ordinate for the swelling percentage (%). In the figure, the curve (1) represents the data for the control group, the curve (2) for the 0.1 $\mu$g polypeptide VI group, the curve (3) for the 1 $\mu$g polypeptide VI group, and the curve (4) for the 10 $\mu$g polypeptide VI group.

Pharmacological Test Example 5: Testing of IL-1$\beta$ derivatives for radiation damage preventing effect

BALB/c mice (9 weeks old) were intraperitoneally given 1 $\mu$g/mouse or 0.3 $\mu$g/mouse of the polypeptide VI 20 hours before irradiation with a lethal dose of X rays. The above mice were subjected to whole body irradiation with X rays at a dose of 850 roentgens using an X-ray irradiation apparatus (MBR-1505R, Hitachi Medico). Thereafter, the animals were checked for survival daily. A group given PBS was used as a control.

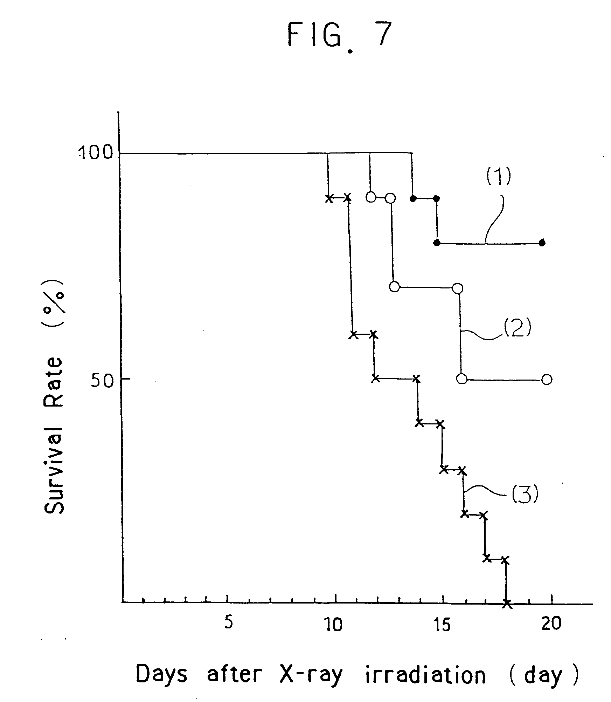

The results are shown in Fig. 7. In the figure, the abscissa is for the number of days after X ray irradiation and the ordinate for the survival rate (%) among the test animals. The curve (1) shows the data for the group given 1 $\mu$g of polypeptide VI, the curve (2) for the group given 0.3 $\mu$g of polypeptide VI, and the curve (3) for the control group.

As seen in Fig. 7, a radiation damage preventing effect was observed in the polypeptide groups in a dose-dependent manner. Whereas, in the control group, all animals were dead on day 18 after X ray irradiation. In the 1 $\mu$g group, it was confirmed that about 80% had avoided death due to radiation damage and had survived.

Pharmacological Test Example 6: Testing of IL-1$\beta$ derivatives for opportunistic infection preventing effect

The following test was carried out using opportunistic infection model mice.

ICR strain male mice (6 weeks old) were used as the test animals (7 animals per group). On day 1, 100 mg/kg of 5-fluorouracil (5-Fu, Kyowa Hakko) was intravenously administered. On days 2, 4 and 6, 1 $\mu$g/mouse of the polypeptide VI was subcutaneously administered and, on day 7, a specified amount of Pseudomona aeruginosa E-2 was intraperitoneally administered for causing infection. On day 10, surviving animals were counted and the survival rate (%) was determined.

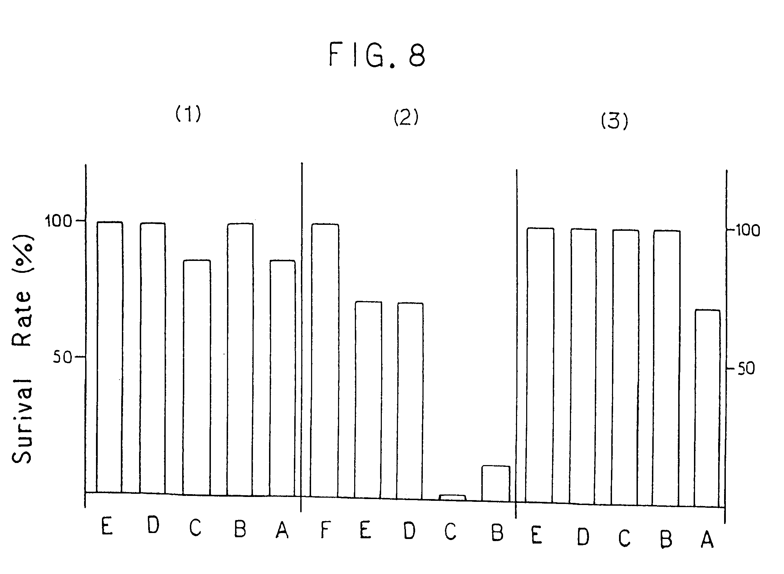

The results are shown in Fig. 8 (1) to (3).

Fig. 8 (1) shows the results in the above experiment group, Fig. 8 (2) shows the results in the polypeptide VI administration-free control group (5-Fu alone was administered), and Fig. 8 (3) shows the results in the control group given neither 5-Fu nor the polypeptide VI.

In Fig. 8, the ordinate is for the survival rate (%) and the abscissa for the groups A to E given the following doses of Pseudomonas aeruginosa, respectively:

Group A ... Group given 19000 cells/mouse,
Group B ... Group given 3800 cells/mouse,
Group C ... Group given 750 cells/mouse,
Group D ... Group given 150 cells/mouse,
Group E ... Group given 30 cells/mouse,
Group F ... Group given 6 cells/mouse.

〈Method of producing cytokines in animal cells〉

(1) HBS-2C5B2 cells [J. Immunol., 131, 1682-1689 (1985)] were cultured in an amount of 2 x $10^5$ cells/well in the presence of various concentrations of the polypeptide XXXVII and 0.01% PHA-P. After 24 hours of incubation, the supernatant was collected and its IL-2 activity was determined by the method of K. A. Smith et al. using IL-2-dependent mouse T cells (CTLL2) [J. Immunol., 120, 2027 (1978)].

The results are shown below in Table 5.

Table 5

| Concentration of polypeptide XXXVII (ng/ml) | IL-2 activity (cpm x $10^3$) Mean (n = 5) |
|---|---|
| 0 | 0.2 |
| 0.0005 | 0.3 |
| 0.005 | 1.3 |
| 0.05 | 3.9 |
| 0.5 | 3.1 |
| 5 | 4.1 |
| 50 | 4.2 |
| 500 | 4.9 |

(2) U-373MG cells were cultured until confluence in RPMI-1640 medium supplemented with 10% FCS and further incubated in the above medium with or without (control) 20 ng/ml of the polypeptide XXXVII for 18 hours.

After removal of the medium, RNA was extracted by the guanidinium/cesium chloride method and subjected to oligo(dT)-cellulose chromatography to give poly(A)+RNA (mRNA). According to the procedure of Northern blotting, the above poly(A)+RNA (10 $\mu$g) was subjected to agarose gel (1.2%) electrophoresis for fractionation, followed by transfer to a nitrocellulose filter. After baking at 80°C under reduced pressure, the filter was treated in 20 mM Tris-HCl (pH 8.0) at 100°C for 5 minutes, followed by prehybridization at 42°C in 50% formamide, 5 x SSC, 50 mM sodium phosphate (pH 6.5), 4 x Denhardt's solution and 200 $\mu$g/ml denatured salmon sperm DNA. After 5 hours, hybridization was

20

conducted at 42°C for 20 hours with the PstI-NcoI DNA fragment of GM-CSF cDNA [Science, 228, 810 (1985)] radiolabeled by nick translation or the KpnI-BamHI DNA fragment of BSF-2 cDNA [Nature, 324, 73 (1986)] radiolabeled in the same manner. The filter was washed with 2 x SSC supplemented with 0.1% SDS at room temperature for 15 minutes and further with 0.1 x SSC supplemented with 0.1% SDS at 50°C for 1 hour. Autoradiography was performed overnight at -70°C using a sensitized paper.

As a result, it was found that natural cytokines can be produced efficiently in animal cells not only with the DNA fragment of GM-CSF but also with the DNA fragment of BSF-2.

The use of such IL-1$\beta$ derivatives in a very slight amount, generally about 10 ng/ml, is sufficient for its application in such method and thus facilitates the process for purifying the cytokine induced.

(3) For the production of cytokines in animal cells, it is essential that the IL-1$\beta$ active substance used for inducing the production should be stable and capable of binding to IL-1 receptors on the cell surface under the induction conditions. Thus, it is important that the IL-1$\beta$ active substance should bind to IL-1 receptors and transmit a signal necessary for cytokine production to the inside of cells. Therefore the following test was performed concerning the binding to IL-1 receptors on fibroblasts. Thus, 50000 cpm/well of the polypeptide I (IL-1$\beta$) labeled with $^{125}$I and 20 ng/ml of the polypeptide I incubated in advance in D-MEM medium supplemented with 10% FCS at 37°C were added to Balb/3T3 cells (clone A31; ATCC, CCL-163; 1 x 10$^6$ cells/well) almost uniformly grown over a 6-well plate all over each well and almost uniformly, and the reaction was allowed to proceed at 4°C. The reaction liquid was removed with a Pasteur pipet, 1 ml of D-MEM supplemented with 10% FCS was added and, after gentle washing, the supernatant was discarded. After two repetitions of this washing procedure, the cells were solubilized with 1 ml of 1% SDS plus 0.2 N NaOH, and the solubilized cell solution, together with an additional portion of the solubilized cell solution used for washing, was subjected to radioactivity (bound radioactivity) counting with a $\gamma$-counter.

The above-mentioned $^{125}$I-labeled polypeptide I was produced and purified by the method of Bolton and Hanter [Biochem. J., 133, 529 (1973)] (specific activity: not less than 250 $\mu$Ci/$\mu$g of protein).

The results obtained are shown below in Table 6.

Table 6

| Incubation period (hrs) | Inhibition (%) |
|---|---|
| 0 | 100 |
| 5 | ca 40 |
| 30 | ca 4 |

In the table, the percent inhibition was calculated as follows:

$$\text{Inhibition (\%)} \quad \frac{A - C}{A - B} \times 100$$

A = Bound radioactivity in the absence of the unlabeled polypeptide I.
B = Radioactivity nonspecifically bound to the plate.
C = Measured value of bound radioactivity.

Such index indicates the binding ability of the conjointly present polypeptide to the I1-1 receptor.

From the above Table 6, it has become apparent that the ability of the polypeptide I, namely IL-1$\beta$ itself, to bind to IL-1 receptors decreases with the lapse of time under cytokine inducing conditions.

Therefore the same test was performed using the polypeptide I, polypeptide VI and polypeptide XXXVII after 24 hours of preliminary incubation.

The results are shown in Table 7 in the same manner as in Table 6.

Table 7

| Polypeptide | Inhibition (%) |
|---|---|
| I | ca 4 |
| VI | ca 44 |
| XXXVII | ca 87 |

The above results indicate that the use of IL-1$\beta$ derivatives is preferred in the production of cytokines in animal cells.

Example 1 - no example according to the present invention

To 0.01 M citric acid-sodium citrate buffer (pH 6.0) there were added the polypeptide VI, Tween 80 (Polysorbate 80; Nippon Surfactant) and maltose in concentrations of 45000 units as GIF activity [determined by the method mentioned above using the cell line A375S1 (Fermentation Research Institute deposit No. 9670) as human melanoma cells] per milliliter (about 1 $\mu$g of protein/ml), 0.01 mg/ml and 15 mg/ml, respectively. The resultant mixture was filtered (with a 0.22 $\mu$m membrane filter), and the filtrate was poured in 1-ml portions into vials aseptically and then lyophilized to give a composition according to the invention in the form of a preparation for injection. Said preparation is applied as dissolved in 1 ml of phyisological saline before use.

Example 2

(1) Injectable compositions according to the invention were prepared in the same manner as described in Example 1 except that the ingredients used were as specified below in items a) to c).

a)

| Polypeptide VI | 10 $\mu$g/ml |
|---|---|
| Tween 80 | 0.01 mg/ml |
| Dextran 40 | 15 mg/ml |
| Cysteine | 0.1 mg/ml |
| HSA | 1 mg/ml |
| (The same buffer solution was used.) | |

b)

| Polypeptide VI | 10 $\mu$g/ml |
|---|---|
| Tween 80 | 0.01 mg/ml |
| Mannitol | 15 mg/ml |
| Cysteine | 0.1 mg/ml |
| HSA | 1 mg/ml |
| (The same buffer solution was used.) | |

c)

| Polypeptide VI | 10 $\mu$g/ml |
|---|---|
| Tween 80 | 0.01 mg/ml |
| Inositol | 15 mg/ml |
| Cysteine | 0.1 mg/ml |
| HSA | 1 mg/ml |
| (The same buffer solution was used.) | |

(2) The preparations prepared as described above in section (1) were tested for stability by the following method.

(I) Method:

The above preparations were stored in glass bottle (airtight and protected from light) containers at 4°C or 25°C and, after 1 week, 2 weeks and 4 weeks of storage, evaluated for the description items (appearance and solution) and content (%) according to the criteria mentioned below.

[Evaluation criteria]

Characteristics: (Appearance) When the initial white lump form was retained, the judgment "no change" was given.
(Solution) When the solution obtained by dissolution in 1 ml of physiological saline was colorless and clear, the judgment "no change" was given.
Hydrogen ion concentration: When it was in the range of initial value (5.65) ± 0.2, the judgment "no change" was given.
Osmotic pressure ratio: When it remained within the range of 95-105%, with the initial value taken as 100%, the judgment "no change" was given.
For the above judgments, the mean of values of 3 vials was evaluated for each sample.

(II) Results:

All the preparations [a) to c) mentioned above] were judged to have undergone "no changes" under all the storage conditions. The measurement data (each being a mean) obtained after 4 weeks of storage are shown below in Table 8.

Table 8

| Preparation tested | Storage temperature | Appearance | Solution | Content (%) |
|---|---|---|---|---|
| a | 4 | White lump | Colorless and clear | 99.6 |
|   | 25 | " | " | 95.0 |
| b | 4 | " | " | 98.6 |
|   | 25 | " | " | 98.9 |
| c | 4 | " | " | 99.6 |
|   | 25 | " | " | 100.3 |

The test results shown above indicate that the compositions according to the invention are very excellent in the stabilization of IL-1$\beta$ substances.

Example 3

(1) A composition according to the invention which had the composition specified below and contained 0.1 $\mu$g/ml or 0.01 $\mu$g/ml of an IL-1$\beta$ active substance [polypeptide VI] was poured into glass vials, silicone-coated glass vials, polypropylene containers and polystyrene containers.

23

⟨Composition formula⟩

| IL-1$\beta$ active substance | 0.1 or 0.01 $\mu$g/ml |
|---|---|
| 0.01 M citrate buffer (pH 6.0) | |
| Sucrose | 5 mg |
| Cysteine | 0.1 mg |
| Human serum albumin | 0.01 to 10 mg/ml |

(2) The compositions according to the invention prepared as described above in section (1) were each subjected to the following adsorption prevention test. Thus, each composition sample was allowed to stand at 4°C for 2 days or 4 days and then measured for the residual IL-1 activity by the following enzyme immunoassay.

⟨Enzyme immunoassay⟩

A mouse anti-IL-1$\beta$ active substance monoclonal antibody is added to a 96-well microplate in an amount of 100 $\mu$l/well and allowed to stand overnight at 4°C. After washing, 400 $\mu$l of 1% bovine serum albumin is added and allowed to stand at room temperature for 30 minutes for causing blocking to proceed. After washing, 100 $\mu$l of each serial dilution of the sample is added and allowed to stand overnight at 4°C, followed by washing. A 100 $\mu$l portion of rabbit anti-IL-1$\beta$ antibody [see Clinica Chimica Acta, vol. 166, pp. 237-246 (1987) and Europ. J. Immunolog., vol. 17, pp. 1527-1530 (1987)] is added thereto and allowed to stand at 37°C for 2 hours. After washing, 100 $\mu$l of a solution of peroxidase-labeled antibody rabbit globulin (Bio-Rad) is added and allowed to stand at 37°C for 2 hours. After washing, 100 $\mu$l of a substrate solution is added and allowed to stand at room temperature for 2 to 15 minutes. The reaction is stopped with 2 N sulfuric acid and the absorbance at 492 nm is measured. Separately, a calibration curve is constructed based on IL-1$\beta$ active substance solutions of known concentrations. The IL-1$\beta$ active substance concentration of the sample is determined from the calibration curve.

The results thus obtained are shown in Table 9.

In Table 9, the numeral 1 in the "Container type*" column stands for glass vials, 2 for silicone-coated glass vials, 3 for polypropylene containers, and 4 for polystyrene containers.

From this Table 9, it is evident that the samples of the compositions according to the invention do not allow adsorption of IL-1$\beta$ on container surfaces.

Table 9

| IL-1β active substance concentration (ng/ml) | HSA concentration (mg/ml) | Container type* | Residual amount (ng/ml) | | |
|---|---|---|---|---|---|
| | | | Initial | After 2 days | After 4 days |
| 100 | 0 | 1 | 101.5± 5.0 | 22.0±11.0 | 3.5± 1.0 |
| | | 2 | | 13.0±22.0 | 7.0±13.0 |
| | | 3 | | 0 | 0 |
| | | 4 | | 0 | 0 |
| 100 | 0.01 | 1 | 103.0±11.5 | 100.0± 6.0 | 96.0±18.0 |
| | | 2 | | 98.5± 5.0 | 111.0± 6.0 |
| | | 3 | | 97.0± 4.0 | 99.0± 8.5 |
| | | 4 | | 105.0±16.5 | 106.0± 9.0 |
| 100 | 0.1 | 1 | 101.5± 9.0 | 111.0± 2.0 | 104.0±11.0 |
| | | 2 | | 105.0±12.0 | 105.0±11.0 |
| | | 3 | | 90.0±16.0 | 108.0± 8.5 |
| | | 4 | | 103.0± 3.0 | 103.0±12.0 |
| 100 | 1.0 | 1 | 114.0±14.0 | 116.0±12.0 | 115.0±25.0 |
| | | 2 | | 93.0±15.0 | 99.0±15.0 |
| | | 3 | − | 118.0±12.0 | 93.0± 6.5 |
| | | 4 | | 97.0±11.5 | 116.5± 8.5 |
| 100 | 10 | 1 | 107.0± 6.0 | 101.0± 6.0 | 95.0±21.0 |
| | | 2 | | 98.0±16.0 | 94.0±18.0 |
| | | 3 | | 101.0±16.0 | 92.0± 5.5 |
| | | 4 | | 97.0± 1.0 | 114.0±11.5 |

| IL-1β active substance concentration (ng/ml) | HSA concentration (mg/ml) | Container type* | Residual amount (ng/ml) | | |
|---|---|---|---|---|---|
| | | | Initial | After 2 days | After 4 days |
| 10 | 0 | 1 | 10.8± 0.6 | 3.2± 1.0 | 1.9± 1.3 |
| | | 2 | | 0 | 1.1± 1.8 |
| | | 3 | | 0.1± 0.2 | 0 |
| | | 4 | | 0 | 0.2± 0.3 |
| 10 | 0.01 | 1 | 10.8± 0.7 | 9.9± 1.4 | 11.3± 0.5 |
| | | 2 | | 11.2± 1.2 | 11.4± 0.2 |
| | | 3 | | 9.1± 1.2 | 9.3± 1.9 |
| | | 4 | | 9.4± 2.7 | 10.3± 0.9 |
| 10 | 0.1 | 1 | 10.1± 0.8 | 11.0± 1.1 | 11.7± 0.9 |
| | | 2 | | 10.3± 0.5 | 9.3± 0.5 |
| | | 3 | | 10.1± 1.0 | 9.9± 1.2 |
| | | 4 | | 9.2± 3.0 | 10.1± 1.7 |
| 10 | 1.0 | 1 | 10.5± 0.7 | 10.3± 1.7 | 10.5± 0.7 |
| | | 2 | | 10.1± 0.9 | 11.4± 0.1 |
| | | 3 | | 10.2± 1.2 | 10.6± 1.5 |
| | | 4 | | 10.7± 3.8 | 10.6± 2.2 |
| 10 | 10 | 1 | 11.1± 0.3 | 9.8± 0.7 | 10.8± 1.0 |
| | | 2 | | 9.9± 1.8 | 11.1± 0.2 |
| | | 3 | | 11.3± 2.6 | 9.8± 0.9 |
| | | 4 | | 10.2± 1.5 | 11.3± 1.6 |

Example 4

(1) To 1 $\mu$g of an IL-1$\beta$ active substance (polypeptide VI) there were added 0.1 mg of human serum albumin, 0.01 M citric acid-sodium citrate buffer (pH 6.0) and the ingredient(s) specified below in Table 10. After mixing, the mixture was filtered (using a 0.22 $\mu$m membrane filter) and the filtrate was poured in 1-ml portions into glass vials under aseptic conditions and then lyophilized to give a composition according to the invention in the form of a pharmaceutical preparation for injection.

EP 0 401 379 B1

Table 10

| Sample No. | Additive(s) | Addition level |
|---|---|---|
| g | Sucrose | 5 mg |
| h | Sucrose<br>Cysteine | 5. mg<br>0.1 mg |
| i | Mannitol<br>Cysteine | 5 mg<br>0.1 mg |
| j | Sucrose<br>Cysteine<br>Isotonizing agent (sodium chloride) | 5 mg<br>0.1 mg<br>8.6 mg |
| k | Sucrose<br>Cysteine<br>Isotonizing agent (glycine) | 5 mg<br>0.1 mg<br>21 mg |
| l | Mannitol<br>Cysteine<br>Isotonizing agent (sodium chloride) | 5 mg<br>0.1 mg<br>8.2 mg |
| m | Sucrose<br>Cysteine<br>Isotonizing agent<br>(citric acid) (Sodium citrate) | 5 mg<br>0.1 mg<br><br>1.5 mg 24.7 mg |

(2) Each sample according to the invention prepared as described above in section (1) was evaluated for its stability in the following manner. Thus, each lyophilized sample was stored in a glass vial (airtight, protected from light) at 25°C, 50°C or 70°C for 1, 2 or 4 weeks (1 week only for storage at 70°C).

The residual IL-1$\beta$ active substance was assayed by chromatography (HPLC; Tosoh HPLC system) as follows:

Column:           TSK $C_{18}$ = NPR (4.6 $\phi$ 35 mm; Tosoh)

Solvents:         Solution A = 0.1% TFA-water

                   Solution B = 0.1% TFA-acetonitrile

Gradient program:

| Time (minutes) | B % |
|---|---|
| 0 | 30 |
| 10 | 36 |
| 13 | 50 |
| 14 | 30 |

Detection:       Ultraviolet absorption (210 nm)

$$\text{Residual \% } = \frac{\text{Residual amount after lapse of predetermined time period}}{\text{Initial amount}} \times 100$$

The results obtained are shown in Table 11.

Table 11

| Sample No. | Residual percentage (%) | | | |
|---|---|---|---|---|
| | Initial | After 1 week | | |
| | | 25°C | 50°C | 70°C |
| o | 100 | 98.4 | 94.1 | 38.3 |
| p | 100 | 96.3 | 95.8 | 76.9 |
| q | 100 | 95.7 | 92.2 | 58.1 |
| r | 100 | 99.7 | 102.7 | 64.6 |
| s | 100 | 95.6 | 100.5 | 63.3 |
| t | 100 | 99.8 | 90.1 | 53.1 |
| u | 100 | 98.5 | 99.5 | 98.2 |

| Sample No. | Residual percentage (%) | | | |
|---|---|---|---|---|
| | After 2 weeks | | After 4 weeks | |
| | 25°C | 50°C | 25°C | 50°C |
| o | 101.1 | 88.3 | 95.3 | 83.3 |
| p | 98.3 | 94.1 | 96.4 | 96.0 |
| q | 95.7 | 91.4 | 95.9 | 78.2 |
| r | 101.7 | 100.7 | 103.5 | 91.0 |
| s | 99.8 | 96.9 | 95.3 | 86.4 |
| t | 95.1 | 87.3 | 96.7 | 65.1 |
| u | 102.1 | 101.3 | 95.5 | 95.7 |

As shown above in Table 11, all the samples of the compositions according to the invention remained unchanged after 4 weeks of storage at 25°C. When stored at 50°C for 4 weeks, the samples of the compositions No. h and m according to the invention did not show any change. When stored at 70°C for 1 week, only the sample of the composition No. m according to the invention did not show any change.

**Claims**

**Claims for the following Contracting States : CH, DE, FR, GB, IT, LI, NL, SE**

1. A stabilized interleukin-1$\beta$ composition which contains:
   (A) an interleukin-1$\beta$ active substance,
   (B) human serum albumin and
   (C) a saccharide.

2. The composition of claim 1, wherein the saccharide is sucrose.

3. The composition of claim 1, which further contains a sulfur-containing reducing agent.

4. The composition of any one of claims 1 to 3, which further contains a citric acid-sodium citrate buffer.

5. The composition of claim 1, wherein the amount of said human serum albumin is 0.1 mg/$\mu$g of the interleukin-1$\beta$ active substance and the amount of said saccharide is 5 mg/$\mu$g of the interleukin 1$\beta$-active substance.

**Claims for the following Contracting State : ES**

1. A method for preparing a stabilized interleukin-1$\beta$ composition which comprises compounding the following components:
   (a) an interleukin-1$\beta$ active substance,
   (b) human serum albumin, and
   (c) a saccharide
   as essential ingredients.

**2.** The method of claim 1 wherein sucrose is used as the saccharide.

**3.** The method of claim 1 which comprises compounding the following components:
   (a) an interleukin-1$\beta$ active substance,
   (b) human serum albumin,
   (c) a saccharide, and
   (d) a sulfur-containing reducing agent.

**4.** The method according to any one of claims 1-3 which comprises compounding the compounds as defined in any one of the claims 1-3 with a citric acid - sodium citrate buffer.

**5.** The method of claim 1 according to which human serum albumin is used in an amount of 0.1 mg/$\mu$g of the interleukin-1$\beta$ active substance and said saccharide is used in an amount of 5 mg/$\mu$g of the interleukin 1$\beta$-active substance.

**6.** A stabilized interleukin-1$\beta$ composition which contains:
   (a) an interleukin-1$\beta$ active substance,
   (b) human serum albumin, and
   (c) a saccharide.

**7.** The composition of claim 6, wherein the saccharide is sucrose.

**8.** The composition of claim 6, which further contains a sulfur-containing reducing agent.

**9.** The composition of any one of claims 6-8, which further contains a citric acid-sodium citrate buffer.

**10.** The composition of claim 6, wherein the amount of said human serum albumin is 0.1 mg/$\mu$g of the interleukin-1$\beta$ active substance and the amount of said saccharide is 5 mg/$\mu$g of the interleukin 1$\beta$-active substance.

**Patentansprüche**
**Patentansprüche für folgende Vertragsstaaten : CH, DE, FR, GB, IT, LI, NL, SE**

**1.** Stabilisierte Interleukin-1$\beta$ Zusammensetzung, enthaltend:
   (A) eine Interleukin-1$\beta$-aktive Substanz,
   (B) Humanserumalbumin und
   (C) ein Saccharid

**2.** Zusammensetzung nach Anspruch 1, wobei das Saccharid Sucrose ist.

**3.** Zusammensetzung nach Anspruch 1, welche weiterhin ein schwefelhaltiges Reduktionsmittel enthält.

**4.** Zusammensetzung nach einem der Ansprüche 1 bis 3, welche weiterhin einen Zitronensäure-Natriumzitratpuffer enthält.

**5.** Zusammensetzung nach Anspruch 1, wobei die Menge des Humanserumalbumins 0,1 mg/$\mu$g Interleukin-1$\beta$-aktive Substanz und die Menge des Saccharids 5 mg/$\mu$g Interleukin-1$\beta$-aktive Substanz beträgt.

**Patentansprüche für folgenden Vertragsstaat : ES**

**1.** Verfahren zum Herstellen einer stabilisierten Interleukin-1$\beta$-Zusammensetzung, umfassend Mischen der nachfolgenden Komponenten:
   (a) eine Interleukin-1$\beta$-aktive Substanz,
   (b) Humanserumalbumin und
   (c) ein Saccharid
   als wesentliche Bestandteile.

**2.** Verfahren nach Anspruch 1, wobei Sucrose als Saccharid verwendet wird.

EP 0 401 379 B1

**3.** Verfahren nach Anspruch 1, umfassend Mischen der folgenden Komponenten:
    (a) eine Interleukin-1$\beta$-aktive Substanz,
    (b) Humanserumalbumin,
    (c) ein Saccharid und
    (d) ein schwefelhaltiges Reduktionsmittel.

**4.** Verfahren nach einem der Ansprüche 1 bis 3, welches Mischen der in einem der Ansprüche 1 bis 3 definierten Verbindungen mit einem Zitronensäure-Natriumzitrat-Puffer umfaßt.

**5.** Verfahren nach Anspruch 1, wobei Humanserumalbumin in einer Menge von 0,1 mg/$\mu$g Interleukin-1$\beta$-aktive Substanz verwendet wird, und das Saccharid in einer Menge von 5 mg/$\mu$g Interleukin-1$\beta$-aktive Substanz verwendet wird.

**6.** Stabilisierte Interleukin-1$\beta$-Zusammensetzung, enthaltend:
    (a) eine Interleukin-1$\beta$-aktive Substanz,
    (b) Humanserumalbumin und
    (c) ein Saccharid.

**7.** Zusammensetzung nach Anspruch 6, wobei das Saccharid Sucrose ist.

**8.** Zusammensetzung nach Anspruch 6, welche weiterhin ein schwefelhaltiges Reduktionsmittel enthält.

**9.** Zusammensetzung nach einem der Ansprüche 6 bis 8, welche weiterhin einen Zitronensäure-Natriumzitrat-Puffer enthält.

**10.** Zusammensetzung nach Anspruch 6, wobei die Menge des Humanserumalbumins 0,1 mg/$\mu$g Interleukin-1$\beta$-aktive Substanz ist, und die Menge des Saccharids 5 mg/$\mu$g Interleukin-1$\beta$-aktive Substanz beträgt.

**Revendications**
**Revendications pour les Etats contractants suivants : CH, DE, FR, GB, IT, LI, NL, SE**

**1.** Composition d'interleukine-1$\beta$ stabilisée, qui contient :
    (A) une substance active interleukine-1$\beta$,
    (B) de la sérum albumine humaine et
    (C) un saccharide.

**2.** Composition suivant la revendication 1, dans laquelle le saccharide est du sucrose.

**3.** Composition suivant la revendication 1, qui comprend de plus un agent réducteur contenant du soufre.

**4.** Composition suivant l'une quelconque des revendications 1 à 3, qui contient de plus un tampon acide citrique-citrate de sodium.

**5.** Composition suivant la revendication 1, dans laquelle la quantité de cette sérum albumine humaine est de 0,1 mg/$\mu$g de la substance active interleukine-1$\beta$ et la quantité de ce saccharide est de 5 mg/$\mu$g de la substance active interleukine-1$\beta$.

**Revendications pour l'Etat contractant : ES**

**1.** Procédé pour la préparation d'une composition d'interleukine-1b stabilisée, qui comprend le mélange des composants suivants :
    (a) une substance active interleukine-1$\beta$,
    (b) de la sérum albumine humaine et
    (c) un saccharide,
comme composants essentiels.

**2.** Procédé suivant la revendication 1, dans lequel du sucrose est utilisé comme saccharide.

30

**3.** Procédé suivant la revendication 1, qui comprend le mélange des composants suivants :
(a) une substance active interleukine-1$\beta$,
(b) de la sérum albumine humaine,
(c) un saccharide, et
(d) un agent réducteur contenant du soufre.

**4.** Procédé suivant l'une quelconque des revendications 1 à 3, qui comprend le mélange des composés tels que définis dans l'une quelconque des revendications 1 à 3 avec un tampon acide citrique-citrate de sodium.

**5.** Procédé suivant la revendication 1, dans lequel la sérum albumine humaine est utilisée en une quantité de 0,1 mg/$\mu$g de la substance active interleukine-1$\beta$ et ce saccharide est utilisé en une quantité de 5 mg/$\mu$g de la substance active interleukine-1$\beta$.

**6.** Composition d'interleukine-1$\beta$ stabilisée, qui contient :
(a) une substance active interleukine-1$\beta$,
(b) de la sérum albumine humaine et
(c) un saccharide.

**7.** Composition suivant la revendication 6, dans laquelle le saccharide est du sucrose.

**8.** Composition suivant la revendication 6, qui comprend de plus un agent réducteur contenant du soufre.

**9.** Composition suivant l'une quelconque des revendications 6 à 8, qui contient de plus un tampon acide citrique-citrate de sodium.

**10.** Composition suivant la revendication 6, dans laquelle la quantité de cette sérum albumine humaine est de 0,1 mg/$\mu$g de la substance active interleukine-1$\beta$ et la quantité de ce saccharide est de 5 mg/$\mu$g de la substance active interleukine-1$\beta$.

# FIG. 1

# FIG. 2

Concentration of Polypeptide I (GIF unit/ml)

# FIG. 3

FIG. 4

EP 0 401 379 B1

# FIG. 5

FIG. 6

EP 0 401 379 B1

# FIG. 7

# FIG. 8

EP 0 401 379 B1